# EUROPÄISCHE PATENTSCHRIFT

(11) **EP 2 376 417 B1**
(45) Veröffentlichungstag und Bekanntmachung des Hinweises auf die Patenterteilung: **19.10.2016**
(21) Anmeldenummer: 09768013.6
(22) Anmeldetag: 30.11.2009
(51) Int. Cl.: C07C 51/42

(54) **VERFAHREN ZUR RÜCKSPALTUNG VON IN EINER FLÜSSIGKEIT F ENTHALTENEN MICHAEL-ADDUKTEN, DIE BEI DER HERSTELLUNG VON ACRYLSÄURE ODER DEREN ESTER GEBILDET WURDEN**
PROCESS FOR REDISSOCIATION OF MICHAEL ADDUCTS CONTAINED IN A FLUID F, WHICH WERE FORMED DURING MANUFACTURING ACRYLIC ACID OR ITS ESTER
PROCEDE DE DECOMPOSITION DES PRODUITS DE L'ADDITION DE MICHAEL COMPRENANT DANS UN FLUIDE F, LEQUELS PRODUITS ETAIENT FORMES PENDANT LA PREPARATION D'ACIDE ACRYLIQUE OU SON ESTER

(30) Priorität: 12.12.2008 DE 102008054587; 12.12.2008 US 122154 P
(43) Veröffentlichungstag der Anmeldung: 19.10.2011
(73) Patentinhaber: BASF SE, 67056 Ludwigshafen am Rhein (DE)
(72) Erfinder: SCHLIEPHAKE, Volker, 67105 Schifferstadt (DE); FRIESE, Thorsten, 68199 Mannheim (DE); BOTT, Klaus, 67071 Ludwigshafen (DE); BLECHSCHMITT, Michael, 67105 Schifferstadt (DE); BLUM, Till, Kuantan 25050 (MY); MÜLLER-ENGEL, Klaus, Joachim, 76297 Stutensee (DE)
(74) Vertreter: BASF IP Association
(86) Internationale Anmeldenummer: PCT/EP2009/066063
(87) Internationale Veröffentlichungsnummer: WO 2010/066601

(56) Entgegenhaltungen:
- EP-A1- 1 452 518
- EP-A1- 1 795 521

## Beschreibung

Vorliegende Erfindung betrifft ein Verfahren zur Rückspaltung von in einer Flüssigkeit F mit einem Gewichtsanteil, bezogen auf das Gewicht der Flüssigkeit F, von ≥ 10 Gew.-% enthaltenen Michael-Addukten, die bei der Herstellung von Acrylsäure oder deren Ester gebildet wurden, in einer Rückspaltvorrichtung, die wenigstens eine Pumpe P, eine Trennkolonne K, die von unten nach oben aus einem Sumpfraum, einem sich an den Sumpfraum anschließenden, trennwirksame Einbauten enthaltenden, Trennraum (trennwirksamen Raum) und einem sich an diesen anschließenden Kopfraum besteht und in der der Druck in der Gasphase von unten nach oben abnimmt, sowie einen indirekten Umlaufwärmeaustauscher UW, der wenigstens einen Sekundärraum und wenigstens einen, von dem wenigstens einen Sekundärraum durch eine materielle Trennwand D getrennten, Primärraum aufweist, umfasst, bei dem man kontinuierlich die Flüssigkeit F mit der Zuführtemperatur T^{Z} an einer Zuführstelle I, die sich oberhalb der untersten trennwirksamen Einbauten in der Trennkolonne K befindet, in die Trennkolonne K hineinführt, und an der tiefstgelegenen Stelle des Sumpfraums der Trennkolonne K mit der Pumpe P kontinuierlich einen Mengenstrom *Ṁ* der durch die trennwirksamen Einbauten in den Sumpfraum ablaufenden Michael-Addukte enthaltenden Flüssigkeit mit einer Temperatur T^{SU} entnimmt, so dass sich im Sumpfraum als Sumpfflüssigkeit ein Stand S der in diesen ablaufenden Flüssigkeit einstellt, der weniger als der halbe Abstand A, gemessen vom tiefstgelegenen Punkt der Trennkolonne K bis zur Unterseite der untersten trennwirksamen Einbaute in der Trennkolonne K, beträgt, während im über diesem Flüssigkeitsstand liegenden restlichen Raum des Sumpfraums ein Gasdruck GD besteht, sowie wenigstens einen Teilstrom I des Mengenstroms *Ṁ* durch den wenigstens einen Sekundärraum des indirekten Umlaufwärmeaustauschers UW hindurchführt und dabei durch indirekten Wärmeaustausch mit einem gleichzeitig durch den wenigstens einen Primärraum des Umlaufwärmeaustauschers UW hindurchgeführten fluiden Wärmeträger auf eine oberhalb der Temperatur T^{SU} liegende Rückspalttemperatur T^{RS} erwärmt, und von dem aus dem wenigstens einen Sekundärraum des Umlaufwärmeaustauschers UW mit der Temperatur T^{RS} herausgeführten Stoffstrom *Ṁ** an einer Zuführstelle II, die sich unterhalb der untersten trennwirksamen Einbaute der Trennkolonne K und oberhalb des Stands S Sumpfflüssigkeit befindet, wenigstens einen Teilstrom II in den Sumpfraum der Trennkolonne K so zurückführt, dass der wenigstens eine Teilstrom II im Sumpfraum der Trennkolonne K nicht auf die Sumpfflüssigkeit gerichtet ist und wenigstens von einem der beiden Ströme *Ṁ, Ṁ* * einen Teilstrom als Reststrom ausschleust, mit der Maßgabe, dass die Rückspalttemperatur T^{RS} so bemessen ist, dass einerseits beim Durchströmen des wenigstens einen Sekundärraums des Umlaufwärmeaustauschers UW wenigstens eine Teilmenge der wenigstens einen im Teilstrom I enthaltenen Michael-Addukte unter Ausbildung der zugehörigen Rückspaltprodukte rückgespalten wird, sowie andererseits der in die Trennkolonne K zurückgeführte wenigstens eine Teilstrom II beim an der Zuführstelle II im Sumpfraum vorherrschenden Gasdruck GD siedet und die sich beim Sieden ausbildende, wenigstens eine Teilmenge der Rückspaltprodukte umfassende, Gasphase dem zum Kopfraum der Trennkolonne K hin in der Trennkolonne K abnehmenden Gasdruck folgend als Rückspaltprodukt enthaltender Gasstrom G in den Kopfraum der Trennkolonne K strömt, und der Gasstrom noch im Kopfraum der Trennkolonne K und/oder aus dem Kopfraum der Trennkolonne K herausgeführt durch direkte und/oder indirekte Kühlung teilweise kondensiert, das dabei gebildete Kondensat wenigstens teilweise als Rücklaufflüssigkeit in die Trennkolonne K rückgeführt und der bei der partiellen Kondensation verbleibende Gasstrom ausgelassen wird.

Acrylsäure ist ein bedeutendes Zwischenprodukt, das beispielsweise im Rahmen der Herstellung von Polymerisatdispersionen (dies auch in Form ihrer Ester mit Alkoholen) sowie Wasser superabsorbierenden Polymerisaten Verwendung findet.

Acrylsäure ist unter anderem durch heterogen katalysierte Gasphasen-Partialoxidation von C₃-Vorläuferverbindungen der Acrylsäure (unter diesem Begriff sollen insbesondere solche chemischen Verbindungen zusammengefasst werden, die formal durch Reduktion von Acrylsäure erhältlich sind; bekannte C₃-Vorläufer von Acrylsäure sind z. B. Propan, Propen, Acrolein, Propionaldehyd und Propionsäure; der Begriff soll aber auch Vorläuferverbindungen der vorgenannten Verbindungen umfassen, wie z. B. Glycerin (von Glycerin ausgehend kann Acrylsäure beispielsweise durch heterogen katalysierte oxidative Dehydratisierung in der Gasphase erzeugt werden; vlg. z. B. EP-A 1 710 227, WO 06/114506 und WO 06/092272)) mit molekularem Sauerstoff an im festen Aggregatzustand befindlichen Katalysatoren bei erhöhter Temperatur erhältlich (z. B. die Deutsche Anmeldung mit dem Aktenzeichen 102007055086.5 und die DE-A 10 2006 062 258).

Aufgrund zahlreicher im Verlauf der katalytischen Gasphasen-Partialoxidation erfolgender Parallel- und Folgereaktionen sowie aufgrund der im Rahmen der Partialoxidation mit zu verwendenden inerten Verdünnungsgase wird bei der katalytischen Gasphasen-Partialoxidation keine reine Acrylsäure, sondern ein Reaktionsgasgemisch (ein Produktgasgemisch) erhalten, das im wesentlichen Acrylsäure, die inerten Verdünnungsgase und Nebenprodukte enthält, aus welchem die Acrylsäure abgetrennt werden muss.

Üblicherweise erfolgt die Abtrennung der Acrylsäure aus dem Reaktionsgasgemisch unter anderem dadurch, dass die Acrylsäure durch Anwendung absorptiver und/oder kondensativer Maßnahmen zunächst aus der Gasphase in die kondensierte (flüssige) Phase überführt wird. Die weitere Abtrennung der Acrylsäure aus der so erzeugten Flüssigphase wird nachfolgend üblicherweise über extraktive, destillative, desorptive, kristallisative und/oder sonstige thermische Trennverfahren vorgenommen. Alternativ kann Acrylsäure auch durch homogen katalysierte Verfahren ausgehend von z. B. Acetylen (z. B. Reppe Prozess) oder Ethylen (Oxycarbonylierung) hergestellt werden. Für die Abtrennung der Acrylsäure aus den dabei resultierenden Reaktionsgemischen gilt das Vorgenannte in entsprechender Weise.
Nachteilig an Acrylsäure und den vorgenannten Trennverfahren ist, dass Acrylsäure in flüssiger Phase befindlich zur Bildung von unerwünschten Nebenprodukten neigt. Eine solche unerwünschte Nebenreaktion ist die radikalische Polymerisation mit sich selbst unter Ausbildung von Acrylsäurepolymerisat bzw. -oligomerisat. Nachteilig an dieser Nebenreaktion ist, dass sie im Wesentlichen irreversibel ist, weshalb in radikalisches Acrylsäurepolymerisat gewandelte monomere Acrylsäure für den Acrylsäureherstellprozess verloren ist und die Acrylsäureausbeute des Herstellverfahrens mindert. Vorteilhaft an der unerwünschten radikalischen Polymerisation von Acrylsäure ist jedoch, dass sie durch Zusatz von Polymerisationsinhibitoren wenigstens gemindert werden kann.

Letzteres trifft auf die zweite unerwünschte Nebenreaktion von Acrylsäure in flüssiger Phase nicht zu. Bei dieser Nebenreaktion handelt es sich um die sogenannte Michael-Addition eines Acrylsäuremoleküls an ein anderes Acrylsäuremolekül, unter Ausbildung eines dimeren Michael-Addukts (Michael-Diacrylsäure), die sich durch weiterführende Michael-Addition (EP 1 795 521) von Acrylsäuremolekülen ("monomerer Acrylsäure") an bereits gebildete Michael-Addukte unter Ausbildung von Michael-Acrylsäureoligomeren fortsetzen kann.
Solche Michael-Addukte lassen sich durch die allgemeine Formel I, 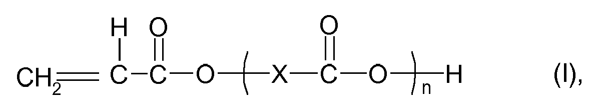 charakterisieren, in der n eine ganze Zahl ≥ 1 ist, und -X- für -CH₂-CH₂- oder für 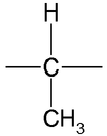 steht, wobei im Fall n ≥ 2 alle X identisch oder auch verschieden sein können. n ist grundsätzlich nicht beschränkt, bewegt sich jedoch in erster Linie im Bereich von 1 bis 15, bzw. von 1 bis 10. Im Beisein von protischen Säuren wie z. B. Wasser sowie bei erhöhter Temperatur erfolgt die Michael-Addition beschleunigt.

Die Herstellung von Estern der Acrylsäure erfolgt großtechnisch überwiegend durch direkte Umsetzung von Acrylsäure mit den entsprechenden Alkoholen in flüssiger Phase.

Allerdings fallen auch in diesem Fall zunächst, in der Regel flüssige, Produktgemische an, aus denen die Acrylsäureester abgetrennt werden müssen. Diese Produktgemische enthalten in der Regel radikalische Polymerisate der Ausgangsacrylsäure und/oder der Produktester als unerwünschte Nebenprodukte. Sie enthalten aber auch Michael-Addukte der allgemeinen Formel (I), die sich aus der Ausgangsacrylsäure in der flüssigen Reaktionsphase in unvermeidbarer Weise bilden.

Aufgrund des Beiseins von freiem Alkohol im flüssigen Veresterungsreaktionsgemisch können die Michael-Addukte der allgemeinen Formel (I) durch Umsetzung (Substitution oder Veresterung) mit solchem freiem Alkohol R-OH (R = organischer Rest) im Produktgemisch einer Acrylsäureveresterung zusätzlich wenigsten in einer gemäß der beiden nachfolgenden allgemeinen Formeln (II) und (III) derivatisierten Form vorliegen, 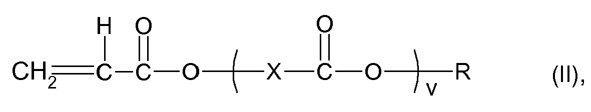 und

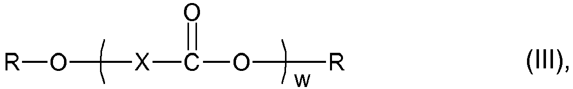

In denen X die gleiche Bedeutung wie in der allgemeinen Formel (I) besitzt und v und w wiederum jeweils für eine ganze Zahl ≥ 1 stehen (in erster Linie liegen v bzw. w im Bereich von 1 bis 15, oder von 1 bis 10).

Verbindungen der allgemeinen Formeln (I), (II) und (III) sollen daher in dieser Schrift unter der Bezeichnung "Michael-Addukte" (die bei der Herstellung von Acrylsäure oder deren Ester gebildet wurden) subsumieren. Fehlt in dieser Schrift das Präfix "Michael", so meinen die Bezeichnungen "Oligomerisat" und "Polymerisat" die durch radikalische Reaktion erwachsenden Verbindungen.

Im Unterschied zur radikalischen Polymerisation der Acrylsäure und/oder ihrer Ester sind die Bildungsreaktionen der Michael-Addukte in typischer Weise reversible Bildungsreaktionen. So ist es bekannt, aus Michael-Addukten (I), (II) und (III) z. B. durch Einwirkung von Wärme die in selbigen chemisch gebundenen Edukte Acrylsäure, Veresterungsalkohol und Ester der Acrylsäure mit dem Veresterungsalkohol wenigstens teilweise rückzubilden (vgl. z. B. DE-A 197 01 737, DE-A 23 39 519, EP-A 1 357 105, EP-A 780 360). Da die Siedepunkte dieser Edukte in der Regel unterhalb jener der zugehörigen Michael-Addukte (aus denen sie rückgebildet wurden) liegen, können die rückgebildeten Edukte durch Überlagerung eines entsprechenden Druckgefälles kontinuierlich aus dem Reaktionsgleichgewicht entfernt und die Rückreaktion so sukzessive vervollständigt werden.

Eine so bewirkte Rückgewinnung der in den Michael-Addukten chemisch gebunden enthaltenen Edukte ist insofern erstrebenswert, als sich dadurch die Zielproduktausbeute bei der Herstellung von Acrylsäure oder ihren Estern erhöhen lässt.

Aufgrund ihrer vergleichweise erhöhten Siedepunkte fallen die Michael-Addukte bei der thermischen Auftrennung von flüssigen Reaktionsproduktgemischen im Rahmen einer Herstellung von Acrylsäure oder deren Ester in der Regel als Bestandteil von Sumpfflüssigkeiten an. In typischer Weise enthalten solche Sumpfflüssigkeiten, bezogen auf ihr Gewicht, ≥ 10 Gew.-% an Michael-Addukten.

Darüber hinaus enthalten solche Michael-Addukte enthaltende Flüssigkeiten neben Acrylsäure und/oder deren Ester üblicherweise noch andere Bestandteile, deren Siedepunkte von jenen der Michael-Addukte verschieden sind.
Diese Siedepunkte können sowohl oberhalb als auch unterhalb jener der Michael-Addukte liegen. Unterwirft man daher Michael-Addukte, die bei der Herstellung von Acrylsäure oder deren Ester gebildet wurden, enthaltende Flüssigkeiten einem Verfahren zur Rückspaltung der in ihnen enthaltenden Michael-Addukte durch Zufuhr thermischer Energie (sowie gegebenenfalls im Beisein geeigneter Rückspaltkatalysatoren, wird das dabei resultierende, wenigstens eine Teilmenge der Rückspaltprodukte enthaltende Spaltgas, vorzugsweise einer Gegenstrom-Rektifikation unterworfen, um die im Spaltgas enthaltenen Rückspaltprodukte mit erhöhter Reinheit rückzugewinnen (vgl. z. B. WO 2004/035514, EP-A 780 360 und DE-A 10 2007 004 960).

Üblicherweise wird daher ein Verfahren zur Rückspaltung von in einer Flüssigkeit mit einem Gewichtsanteil, bezogen auf das Gewicht der Flüssigkeit, von ≥ 10 Gew.-% enthaltenen Michael-Addukten, die bei der Herstellung von Acrylsäure oder deren Ester gebildet wurden, in einer Rückspaltvorrichtung durchgeführt, wie sie in der Präambel dieser Schrift beschrieben ist.

Als Pumpe P wird dabei normalerweise eine Radial-Kreiselpumpe mit geschlossenem Laufrad eingesetzt (vgl. DE-A 102 28 859). Das Prinzip einer Kreiselpumpe erläutert das nachfolgende Beispiel.

Rührt man mit einem Löffel in einem mit Wasser gefüllten Glas, so sinkt der Druck im Zentrum und die Flüssigkeit steigt am Rand des Glases durch den dort herrschenden höheren Druck nach oben.

Je schneller man rührt, desto höher steigt die Flüssigkeit. Hätte das Glas eine seitliche Bohrung oder einen Überlauf oberhalb des ursprünglichen Pegels, dann würde dort Wasser ausfließen. Bei einer Kreiselpumpe übernimmt die Funktion des Löffels ein mit Förderschaufeln ausgestattetes rotierendes Laufrad. Die zu fördernde Flüssigkeit, die in die Pumpe eintritt, wird von den Förderschaufeln des rotierenden Laufrads mitgerissen und dadurch zunächst auf eine Kreisbahn gezwungen. Auf dieser Bahn strömt das dabei durch Impulsübertragung beschleunigte Fluid radial nach außen, wo es durch den Ablauf abfließt. Durch Erweiterung des Strömungsquerschnitts kann die erhöhte Geschwindigkeit proportional in Druck umgewandelt werden. Wird die zu fördernde Flüssigkeit durch das Laufrad im Wesentlichen parallel zur Achse der Laufradantriebswelle befördert, spricht man von einer Kreiselpumpe mit axialem Laufrad, oder auch von einer Axialkreiselpumpe.

Wird die zu fördernde Flüssigkeit durch das Laufrad im Wesentlichen quer zur Achse der Laufradantriebswelle gefördert (radiale Strömung), spricht man von einer Kreiselpumpe mit radialem Laufrad, oder auch von einer Radialkreiselpumpe.

Da bei der Radialkreiselpumpe die zu fördernde Flüssigkeit radial, also senkrecht zur Antriebswelle aus dem Laufrad austritt, erfordert das Wirkungsprinzip der Radialkreiselpumpe im Gegensatz zur Axialkreiselpumpe eine Umlenkung des Förderstroms. Durch diese Strömungsumlenkung werden im Laufrad höhere Zentrifugenkräfte realisiert, die zu höheren Förderdrucken führen. Werden bei einem radialen Laufrad die Laufradschaufeln auf beiden Seiten (oben und unten) mit je einer Scheibe verbunden (Deckscheibe und Tragscheibe), so spricht man von einem geschlossenen radialen Laufrad. Das Verschließen des radialen Laufrads erhöht den hydraulischen Wirkungsgrad der Radialkreiselpumpe und stabilisiert das Laufrad im Betrieb. Aufgrund dieser Vorteile ist die Radialkreiselpumpe mit geschlossenem radialen Laufrad die zur Förderung von Flüssigkeiten meist eingesetzte Pumpe.

Als nachteilig an der Verwendung einer Radialkreiselpumpe in einem Verfahren gemäß der Präambel dieser Schrift hat sich jedoch erwiesen, dass sie keine stabile Langzeitförderleistung gewährleistete. Vielmehr brach die Förderleistung von Zeit zu Zeit in unsystematischer Weise ein.

Die Aufgabe der vorliegenden Erfindung bestand daher darin, ein verbessertes Rückspaltverfahren zur Verfügung zu stellen.

Demgemäß wird ein Verfahren zur Rückspaltung von in einer Flüssigkeit F mit einem Gewichtsanteil, bezogen auf das Gewicht der Flüssigkeit F, von ≥ 10 Gew.-% (von wenigstens 10 Gew.-%) enthaltenen Michael-Addukten, die bei der Herstellung von Acrylsäure oder deren Ester gebildet wurden, in einer Rückspaltvorrichtung, die wenigstens eine Pumpe P, eine Trennkolonne K, die von unten nach oben aus einem Sumpfraum, einem sich an den Sumpfraum anschließenden, trennwirksame Einbauten enthaltenden Trennraum und einen sich an diesen anschließenden Kopfraum besteht und in der der Druck in der Gasphase von unten nach oben abnimmt, sowie einen indirekten Umlaufwärmeaustauscher UW ("Spaltreaktor"), der wenigstens einen Sekundärraum und wenigstens einen, von dem wenigstens einen Sekundärraum durch eine materielle Trennwand D getrennten, Primärraum aufweist, umfasst, bei dem man kontinuierlich die Flüssigkeit F mit der Zuführtemperatur T^{Z} an einer Zuführstelle I, die sich oberhalb der untersten trennwirksamen Einbaute in der Trennkolonne K befindet, in die Trennkolonne K hineinführt, und an der tiefstgelegenen Stelle des Sumpfraums der Trennkolonne K mit der Pumpe P kontinuierlich einen Mengenstrom *Ṁ* der durch die trennwirksamen Einbauten in den Sumpfraum ablaufenden, Michael-Addukte enthaltenden Flüssigkeit mit einer Temperatur T^{SU} entnimmt, so dass sich im Sumpfraum als Sumpfflüssigkeit ein Stand S der in diesen ablaufenden Flüssigkeit einstellt, der weniger als der halbe Abstand A, gemessen vom tiefstgelegenen Punkt der Trennkolonne K bis zur Unterseite der untersten trennwirksamen Einbaute in der Trennkolonne K, beträgt, während im über diesem Flüssigkeitsstand liegenden restlichen Raum des Sumpfraums ein Gasdruck GD besteht, sowie wenigstens einen Teilstrom I des Mengenstroms *Ṁ* durch den wenigstens einen Sekundärraum des indirekten Umlaufwärmeaustauschers UW hindurchführt und dabei durch indirekten Wärmeaustausch mit einem gleichzeitig durch den wenigstens einen Primärraum des Umlaufwärmeaustauschers UW hindurchgeführten fluiden Wärmeträger auf eine oberhalb der Temperatur T^{SU} liegende Rückspalttemperatur T^{RS} erwärmt, und von dem aus dem wenigstens einen Sekundärraum des Umlaufwärmeaustauschers UW mit der Temperatur T^{RS} rückgeführten Stoffstrom *Ṁ** an einer Zuführstelle II, die sich unterhalb der untersten trennwirksamen Einbaute der Trennkolonne K und oberhalb des Stands S (des Flüssigkeitspegels (des Flüssigkeitsspiegels)) der Sumpfflüssigkeit (der in den Sumpfraum der Trennkolonne K ablaufenden Flüssigkeit) befindet, wenigstens einen Teilstrom II in den Sumpfraum der Trennkolonne K so zurückführt, dass der wenigstens eine Teilstrom II im Sumpfraum der Trennkolonne K nicht auf die Sumpfflüssigkeit gerichtet ist, und wenigstens von einem der beiden Ströme *Ṁ, Ṁ* * einen Teilstrom als Reststrom ausschleust, mit der Maßgabe, dass die Rückspalttemperatur T^{RS} so bemessen ist, dass einerseits beim Durchströmen des wenigstens einen Sekundärraums des Umlaufwärmeaustauschers UW wenigstens eine Teilmenge der im wenigstens einen Teilstrom I enthaltenen Michael-Addukte rückgespalten wird, sowie andererseits der in die Trennkolonne K zurückgeführte wenigstens eine Teilstrom I beim an der Zuführstelle II im Sumpfraum vorherrschenden Gasdruck GD siedet und die sich beim Sieden ausbildende, wenigstens eine Teilmenge der Rückspaltprodukte umfassende, Gasphase dem zum Kopf der Trennkolonne K hin in der Trennkolonne K abnehmenden Gasdruck folgend als Rückspaltprodukt enthaltender Gasstrom G in den (zum) Kopfraum der Trennkolonne K strömt, und der Gasstrom G noch im Kopfraum der Trennkolonne K und/oder aus dem Kopfraum der Trennkolonne K herausgeführt durch direkte und/oder indirekte Kühlung teilweise kondensiert, das dabei gebildete Kondensat wenigstens teilweise als Rücklaufflüssigkeit in die Trennkolonne K rückgeführt und der bei der partiellen Kondensation verbleibende Gasstrom ausgelassen wird, zur Verfügung gestellt, das dadurch gekennzeichnet ist, dass die Pumpe P eine Radialkreiselpumpe mit einem halboffenen radialen Laufrad ist.

Unter einem halboffenen radialen Laufrad wird ein radiales Laufrad verstanden, das nur eine Tragscheibe und keine Deckscheibe aufweist. Das heißt, bei einem halboffenen Laufrad sind die Laufradschaufeln nur auf einer Seite mit einer Scheibe verbunden.

Radialkreiselpumpen mit geschlossenem oder mit halboffenem (radialem) Laufrad sind z. B. beschrieben in Strömungsmaschinen, 5. Auflage, Teubner Verlag (2006) und in Pumpen in der Feuerwehr, Teil I, Einführung in die Hydromechanik, Wirkungsweise der Kreiselpumpen, 4. Auflage 1998, Verlag W. Kohlhammer, Berlin.

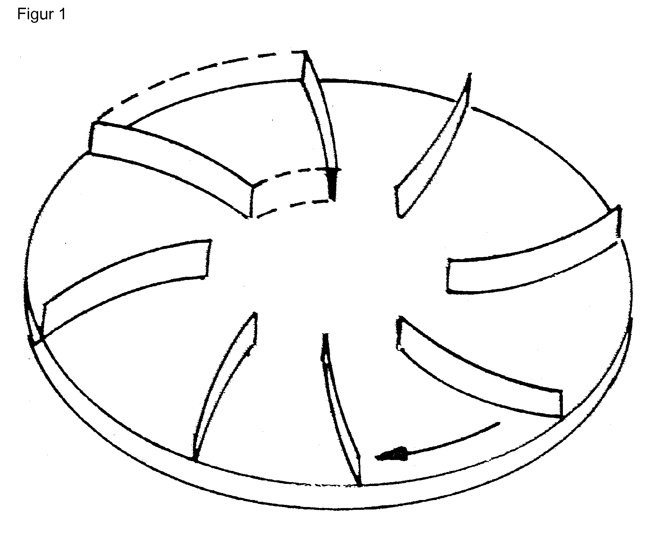

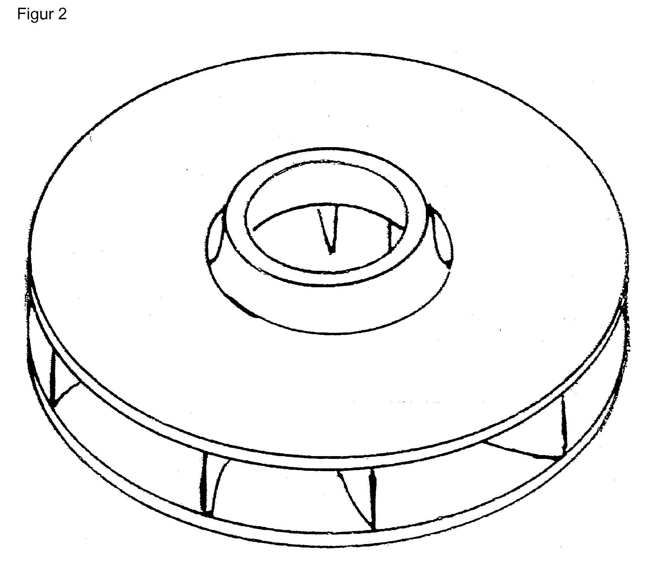

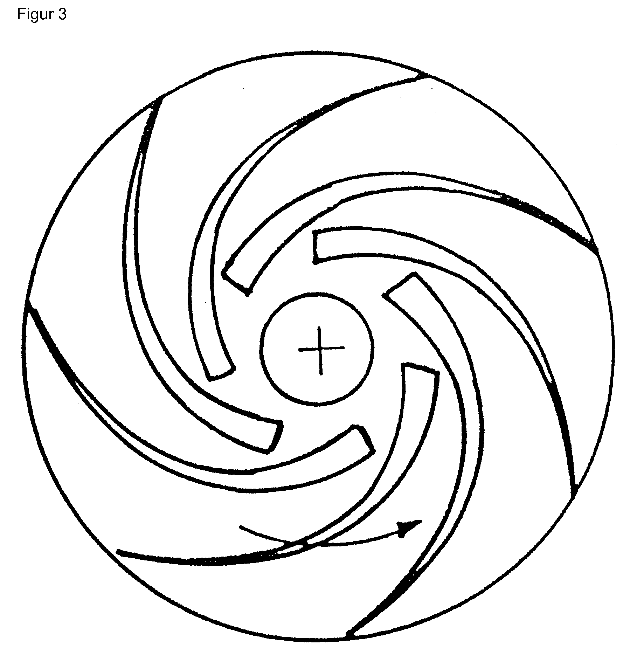

Wie bereits beschrieben, wird bei einer Radialkreiselpumpe mit halboffenem radialem Laufrad durch das sich drehende Laufrad (des mit der Antriebswelle verbundene Förderelement) Arbeit in Form von Bewegungsenergie vom Laufrad auf die zu fördernde Flüssigkeit übertragen. Die Bewegungsenergie wird nach dem Laufrad z. B. in einem Leitrad und/oder im Spiralgehäuse der Pumpe zum überwiegenden Teil wieder in statischen Druck (Druckenergie, Gesetz von der Erhaltung der Energien) umgewandelt. Vom Prinzip her ist ein halboffenes radiales Laufrad eine einfach Scheibe (die Tragscheibe) auf der Schaufeln angebracht sind, wie es die Figur 1 beispielhaft zeigt. Durch die Schaufeln entstehen Schaufelkanäle, deren Querschnitt sich normalerweise von innen nach außen auf Grund des größer werdenden Umfangs sehr stark vergrößert (siehe gestrichelte Linie in Figur 1). Durch diese Schaufelkanäle lässt sich soviel zu fördernde Flüssigkeit wegschleudern, wie in der Mitte des Laufrads zufließen kann. Im Unterschied zum halb offenen Laufrad, das in Figur 1 beispielhaft gezeigt ist, werden beim geschlossenen radialen Laufrad die Schaufelkanäle in einfacher Weise durch eine zweite Scheibe (die Deckscheibe) abgedeckt, die in der Mitte eine Öffnung aufweist (siehe z. B. Figur 2). Die Draufsicht eines halboffenen radialen Schaufellaufrads (Laufrads) zeigt beispielhaft die Figur 3. Die Schaufelkrümmung verläuft in der Regel so, wie die natürliche Bahn eines Wassertropfens auf einer rotierenden runden, glatten Tragscheibe aus der Sicht eines mitrotierenden Beobachters, wenn man den Wassertropfen auf die Scheibenmitte fallen lässt. Diese Schaufelform wird als "rückwärtsgekrümmte" Schaufel bezeichnet.

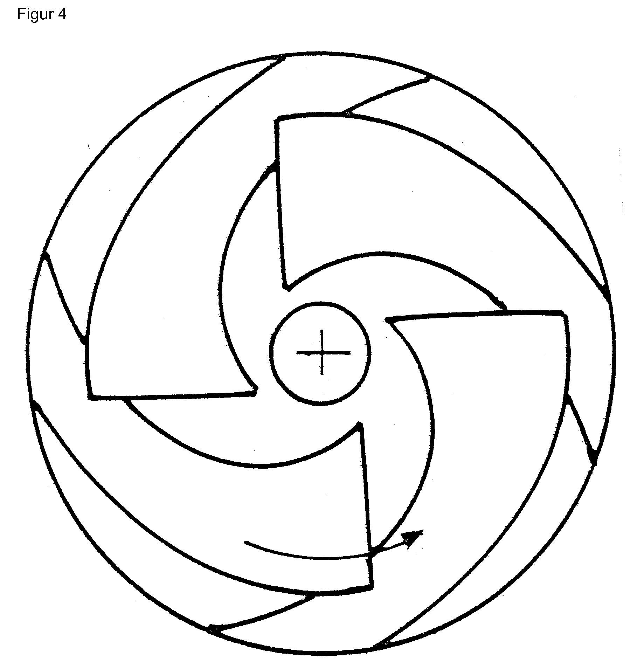

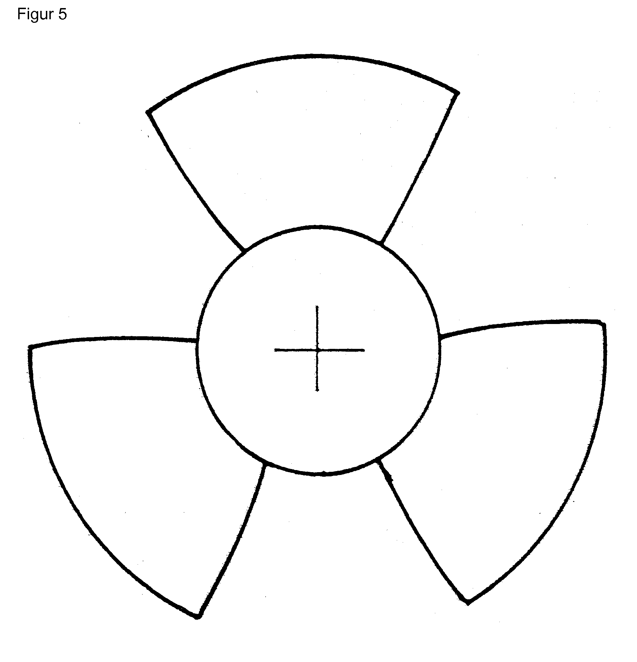

Es können prinzipiell aber auch bis leicht vorwärtsgekrümmte Schaufeln (z. B. wie in Figur 4) und auch schraubenförmige, d. h., in sich verdrehte, rückwärtsgekrümmte Schaufeln, die mit ihren Schneiden bis in den Laufradeintritt hineinragen und die zu fördernde Flüssigkeit wie eine Schiffsschraube erfassen, verwendet werden (vgl. z. B. Figur 5).

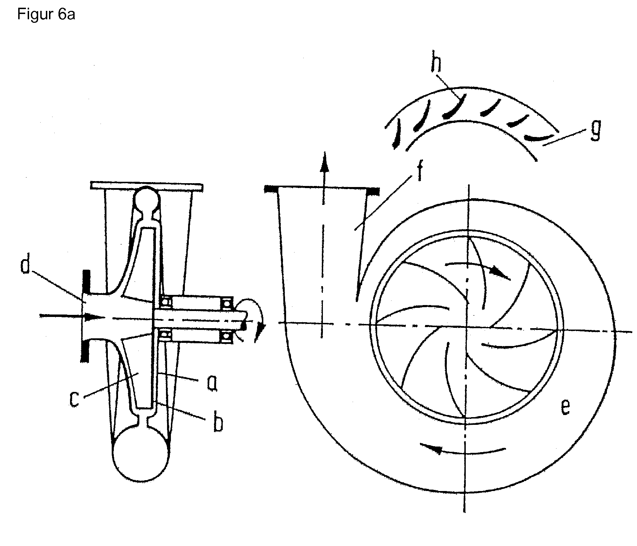

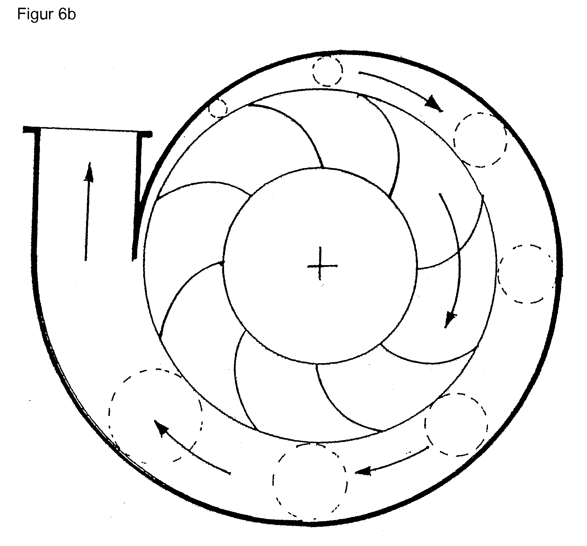

Die Funktionsweise einer Radialkreiselpumpe mit halboffenem radialem Laufrad verdeutlichen die Figuren 6a, 6b beispielhaft.

Die gezeigte Radialkreiselpumpe besteht aus dem Pumpengehäuse (a) und dem in selbigem rotierenden halboffenen Laufrad (b), das mit Schaufeln (c) versehen ist. Die zu fördernde Flüssigkeit tritt axial durch den Saugstutzen (d) ein. Sie wird durch die Fliehkraft radial nach außen gelenkt und vom Laufrad auf diesem Weg auf hohe Geschwindigkeit beschleunigt. Das Pumpengehäuse hat die Aufgabe, die zu fördernde Flüssigkeit von allen Schaufelkanälen aufzufangen, damit diese durch die Druckausgänge (f) weitergeleitet werden kann. Das Pumpengehäuse hat aber gleichzeitig die Aufgabe, Bewegungsenergie der Flüssigkeit in Druck umzuwandeln. Dazu wird in der Regel ausgenutzt, dass eine Querschnittsvergrößerung die Geschwindigkeit der Flüssigkeit herabsetzt und dadurch einen Druckanstieg bewirkt. Zur Querschnittsvergrößerung sind zwei konstruktive Ausführungen des Pumpengehäuses üblich. Als eine Ausführungsform kommen häufig Spiralgehäuse zur Anwendung.

Ein solches umschließt das Laufrad in Spiralform (e). Der Querschnitt erweitert sich in Richtung auf den Druckausgang (siehe zunehmende Kreisradien in Figur 6b).

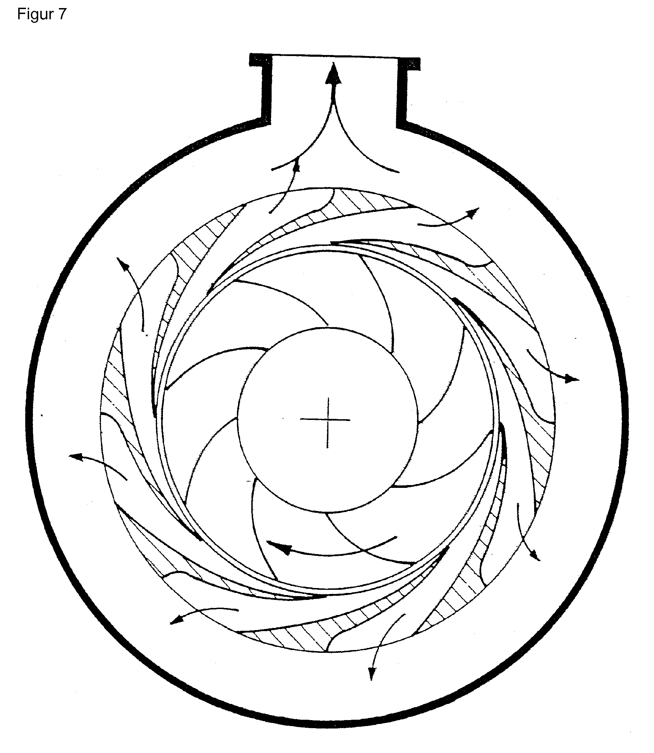

Die durchfließende Flüssigkeit wird dadurch verlangsamt, was eine gleichzeitige Druckzunahme bedeutet. Anstelle der Spirale verwendet man, besonders bei mehrstufigen Pumpen, auch feststehende Leiträder (g). Das Leitrad ist im Pumpengehäuse eingebaut und als Ringraum ausgebildet. Es umschließt das Laufrad. Im Leitrad sind Leitschaufeln (h) angeordnet, die zueinander sich nach außen hin stetig erweiternde Kanäle bilden (vgl. auch Figur 7 in der Draufsicht). Bei dieser Ausführung wird die Flüssigkeit nicht direkt in das Pumpengehäuse geschleudert, sondern sie durchfließt zunächst die Schaufelkanäle des Leitrads. Durch die Erweiterung in Fließrichtung bewirken diese wiederum eine Verlangsamung der Fließgeschwindigkeit und den dadurch bedingten Druckaufbau. Die Richtung der Leitradkanäle ist der Richtung der Laufradkanäle normalerweise entgegengesetzt und entspricht am inneren Umfang des Leitrads der Richtung der Austrittsgeschwindigkeit der Förderflüssigkeit aus dem Laufrad. Selbstverständlich kann auch eine Kombination von Laufrad und Spiralgehäuse angewendet werden. Das heißt, die zu fördernde Flüssigkeit wird erst im Leitrad gesammelt, bevor sie ins Spiralgehäuse gelangen kann.

Während bisher im Wesentlichen nur der Pumpraum beschrieben wurde, soll nachfolgend noch auf den Antriebsraum eingegangen werden. Schnelllaufende Kraftmaschinen wie z. B. Elektromotoren, Verbrennungsmotoren oder Dampfturbinen treiben das Laufrad in direkter Kupplung an. Die Kupplung wird durch eine Antriebswelle bewerkstelligt. Deren Lagerung kann ausschließlich im Antriebsraum untergebracht sein, wie dies die Figur 3 der EP-A 1 092 874 zeigt. Erfindungsgemäß bevorzugt sind der Pumpraum und der Antriebsraum durch einen Trennraum voneinander getrennt.

Mit Vorteil ist der Trennraum mit einem Sperrmedium gefüllt, das aus einem Sperrgas und/oder aus einer Sperrflüssigkeit besteht und von der zu fördernden Flüssigkeit stofflich verschieden oder mit dieser stofflich identisch sein kann. Mit weiterem Vorteil erfolgt keine Lagerung der Antriebswelle innerhalb des Pumpraums. Der Druck des Sperrmediums ist normalerweise größer als der Druck im Pumpraum und als der Druck im Antriebsraum. Ferner ist es anwendungstechnisch zweckmäßig, wenn der durch den Trennraum führende Abschnitt der Antriebswelle sowohl zum Pumpraum als auch zum Antriebsraum hin jeweils mit der Antriebswelle fest und undurchlässig verbundene Gleitelemente trägt, die auf den durch die Antriebswelle durchstoßenen Innenwänden des Trennraums abdichtend gleiten (Prinzip der doppelt (auf beiden Seiten) wirkenden Gleitelement (z. B. Ring)dichtung).

In der Regel ist der Druck im Trennraum wenigstens 1 bar größer, als der Druck an der dem Gleitelement gegenüberliegende Stelle im Pumpraum. Häufig beträgt dieser Druckunterschied > 2 bar, oder > 3 bar. Üblicherweise wird dieser Druckunterschied ≤ 10 bar betragen. Wird als Sperrmedium beim erfindungsgemäßen Verfahren ein Gas verwendet, so handelt es sich bevorzugt um ein Sauerstoff enthaltendes Gas, da molekularer Sauerstoff auf Acrylsäure und ihre Ester polymerisationsinhibierend wirkt.

Diese polymerisationsinhibierende Wirkung entfaltet sich insbesondere im Zusammenhang mit den in der Flüssigkeit F üblicherweise enthaltenen Polymerisationsinhibitoren wie z. B. Phenothiazin oder Methoxyphenol. Selbstverständlich kann die Flüssigkeit F beim erfindungsgemäßen Verfahren aber auch jeden anderen bekannten Polymerisationsinhibitor enthalten.

Bevorzugt beträgt der Sauerstoffgehalt eines solchen Sperrgases 3 bis 21 Vol.-% (Beispielsweise kann auch Kreisgas als Sperrgas verwendet werden).

Bei erfindungsgemäß zu fördernden Flüssigkeiten, deren Flammpunkt (bestimmt nach DIN EN 57) < 50 °C beträgt, ist ein Sauerstoffgehalt eines Sperrgases von 4 bis 10 Vol.-% ganz besonders bevorzugt. Wird beim erfindungsgemäßen Verfahren eine Sperrflüssigkeit verwendet (z. B. 2-Ethylhexanol), so wird diese vorzugsweise so gewählt, dass sie mit der zu fördernden Flüssigkeit verträglich ist.

Für das erfindungsgemäße Verfahren bevorzugte Sperrflüssigkeiten sind Mischungen aus Ethylenglykol und Wasser oder die beiden Flüssigkeiten für sich.

Besonders bevorzugt sind dabei solche Mischungen, deren Gehalt an Ethylenglycol 30 bis 40 Gew.-% beträgt. Die angesprochenen Ethylenglycol-/Wasser-Mischungen zeigen ein ausgezeichnetes Viskositätsverhalten und zeigen sich darüber hinaus unter üblichen Außenbedingungen vergleichsweise gefrierresistent. Im Rahmen des erfindungsgemäßen Verfahrens sind Sperrflüssigkeiten gegenüber Sperrgasen bevorzugt. Als abdichtend wirkende Gleitelemente enthalten sie in der Regel Gleitringdichtungen. Diese bestehen aus einem mit der Antriebswelle fest verbundenen und mit Antriebswelle umlaufenden Gleitring und einem in der Trennrauminnenwand feststehenden Gleitring.

Eine Feder drückt den Gleitring normalerweise mit einer Vorspannung von 1 bis 2 bar gegen den Gegenring. Sie wird im Betrieb um den erhöhten Druck des zwischen den Gleitringdichtungen befindlichen Sperrmediums ergänzt. Durch den erhöhten Druck des Sperrmediums im Vergleich zum auf der Druckseite der Pumpe herrschenden Druck wird ein Austreten der zu fördernden Flüssigkeit aus dem Pumpraum verhindert.

Infolge des erhöhten Drucks im Sperrmedium tritt normalerweise stetig etwas Sperrmedium in die geförderte Flüssigkeit. Im Fall einer Sperrflüssigkeit kann diese Leckrate 0,2 bis 5 ml/h betragen, bei 1 m³/h bis 4000 m³/h an Förderstrom. Für Sperrgase beträgt die Leckrate bezogen auf den gleichen Förderstrom 12 bis 150 Nml/h Sperrgas. Aus Vorratsbehältern wird die Leckrate erfindungsgemäß zweckmäßig kontinuierlich ergänzt.

Das Sperrmedium, z. B. die Sperrflüssigkeit, kann so auch zur Schmierung der Gleitflächen beitragen. Nähere Angaben zur Berechnung und Konstruktion von axialen Gleitringdichtungen finden sich in E. Mayer: Berechnung und Konstruktion von axialen Gleitringdichtungen, Konstruktion 20, 213 - 219 (1968). Es sei an dieser Stelle noch festgehalten, dass in dieser Schrift unter einem Lager ganz allgemein ein Maschinenelement zum Tragen oder Führen von relativ zueinander beweglichen Maschinenteilen verstanden werden soll, wobei es die auftretenden Kräfte aufnimmt und auf das Gehäuse, Bauteil oder Fundament ableitet.

Eine für das erfindungsgemäße Verfahren in besonders vorteilhafter Weise geeignete Radialkreiselpumpe ist die Radialkreiselpumpe KSB CPKN-C1.V 200 - 400, der KSB Aktiengesellschaft in D-67227 Frankenthal (Pfalz), mit doppelt wirkender Gleitringdichtung und einem Ethylenglycol/Wasser-Gemisch als Sperrflüssigkeit.

Allerdings wird vorgenannte Radialkreiselpumpe in ihrer Standardausführung nur mit geschlossenem radialem Laufrad geliefert. Für einen erfindungsgemäßen Einsatz dieser Pumpe muss daher zuvor die Deckscheibe von den Laufradschaufeln entfernt werden (CPK = Chemienormpumpe; N = verstärkter Lagerstuhl zur verbesserten Lagerung der Antriebswelle; C1 = DIN Werkstoff 1.4408, V = VDMA Werkstoff-Schlüssel, 200 = Nennweite Druckseite in mm, 400 = Laufraddurchmesser in mm).

Das erfindungsgemäße Verfahren eignet sich unter anderem im Fall von Flüssigkeiten F, die Michael-Addukte enthalten, die bei der Herstellung von Estern der Acrylsäure mit ein- oder zweiwertigen (eine oder zwei Hydroxylgruppen aufweisenden) Alkoholen (z. B. C₁- bis C₁₀-, bzw. bis C₈-Alkoholen), insbesondere Alkanolen, gebildet werden (durch direkte Umsetzung der Acrylsäure mit dem jeweiligen Alkohol). Dies trifft insbesondere dann zu, wenn es sich beim jeweiligen Alkohol um ein C₁- bis C₁₀-Alkanol (z. B. um ein C₄- bis C₁₀-, oder um C₄- bis C₈-Alkanol) handelt. Zu diesen Estern der Acrylsäure gehören z. B. Hydroxyethylacrylat, 4-Hydroxybutylacrylat, Hydroxypropylacrylat, Methylacrylat, n-Butylacrylat, iso-Butylacrylat, tert.-Butylacrylat, Ethylacrylat, 2-Propylheptylacrylat sowie 2-Ethylhexylacrylat.

Das erfindungsgemäße Verfahren ist aber auch anwendbar im Fall von Flüssigkeiten F, die Michael-Addukte enthalten, die bei der Herstellung von Estern der Acrylsäure mit Aminoalkoholen wie z. B. Aminoethanol, N-Methylaminoethanol und N,N-Dimetylaminoethanol gebildet werden (durch direkte Umsetzung der Acrylsäure mit dem jeweiligen Alkohol).

Derartige Michael-Addukte enthaltende Flüssigkeiten F sind z. B. in den Schriften EP-A 780 360, DE-A 197 01 737, DE-A 2339519, DE-AS 1279015 und EP-A 1 357 105 (einschließlich ihrer Entstehung) offenbart.

In einfacher Weise fallen sie z. B. an, wenn man bei einer in der Regel säurekatalysierten Veresterung von Acrylsäure mit Alkoholen (z. B. einwertigen Alkanolen) vom dabei resultierenden Produktgemisch die nicht umgesetzten Ausgangsstoffe und den gebildeten Acrylester, die im Vergleich zu den relevanten Michael-Addukten normalerweise leichter flüchtig sind, rektifikativ abtrennt, so dass sich die Michael-Addukte im Rektifikationssumpf anreichern und diese Sumpfflüssigkeit eine typische, nach ihrer Entnahme aus der Rektifikationskolonne erfindungsgemäß zu behandelnde Flüssigkeit F bildet. Dabei kann die Veresterung sowohl in einem Lösungsmittel als auch ohne Lösungsmittelzusatz durchgeführt worden sein.

In der Regel enthalten erfindungsgemäß zu behandelnde Flüssigkeiten F, auch diejenigen, deren in ihnen enthaltene Michael-Addukte bei der Herstellung von Acrylsäure gebildet wurden, wenigstens (≥) 20 Gew.-%, oder wenigstens 30 Gew.%, oder wenigstens 40 Gew.-% (jeweils ihres Gewichts) an Michael-Addukten. In der Regel liegt der vorstehende Gehalt an Michael-Addukten in erfindungsgemäß zu behandelnden (einem erfindungsgemäßen Verfahren zu unterwerfenden) Flüssigkeiten F jedoch bei ≤ 90 Gew.-%, meist ≤ 80 Gew.-% und oft ≤ 70 Gew.-% oder sogar ≤ 60 Gew.-% (die Analyse von erfindungsgemäß zu behandelnden Flüssigkeiten F erfolgt in der Regel mittels GC und HPLC).

In typischer Weise kann eine einem erfindungsgemäßen Verfahren zu unterwerfende Flüssigkeit F, deren in ihr enthaltene Michael-Addukte bei der Herstellung eines Esters der Acrylsäure gebildet wurden (z. B. eines C₁- bis C₁₀- bzw. bis C₈-Alkanols bzw. -Alkohols oder eines C₄- bis C₈-Alkanols bzw. -Alkohols, oder eines C₁ bis C₄-Alkanols bzw. -Alkohols) z. B. die folgenden Gehalte aufweisen:

| | |
|---|---|
| 1 bis 30 Gew.-% | Acrylsäureester, |
| 40 bis 80 Gew.-% | Michael-Addukte, und als Restmenge im wesentlichen Polymerisationsinhibitor (typisch 0,1 bis 2 Gew.-%) und radikalisches Polymerisat (in der Regel wenigstens 15 Gew.-%) der Acrylsäure und des Acrylsäureesters. |

In der Regel bestehen die Michael-Addukte dabei zu wenigstens 50 Gew.-% ihres Gesamtgewichtes aus Michael-Addukten der allgemeinen Formel III.

Häufig wird einer einem erfindungsgemäßen Verfahren zu unterwerfenden Flüssigkeit F vorab der Rückspaltung noch Spaltkatalysator zugesetzt, um diejenige Temperatur zu erniedrigen, bei der bereits in nennenswertem Umfang eine Rückspaltung erfolgt. Als solche Rückspaltkatalysatoren kommen z. B. Mineralsäuren wie z. B. Schwefelsäure und Phosphorsäure, von Acrylsäure verschiedene organische Säuren wie z. B. Alkyl- oder Arylsulfonsäuren wie Methansulfonsäure oder p-Toluolsulfonsäure in Betracht. Auch können Amine zugesetzt werden, wie es z. B. die DE-A 10 2006 062 258 beschreibt. Grundsätzlich können beim erfindungsgemäßen Verfahren alle in der Literatur bekannten (vgl. z. B. WO 2004/035514, EP-A 780 360, EP-A 1 357 105, DE-A 2339519, DE-AS 1 279 015, WO 2004/035514, JP-A 178949, DE-A 197 01 737) Spaltkatalysatoren mitverwendet werden.

Bezogen auf ihr an Spaltkatalysatoren freies Gewicht kann die Flüssigkeit z. B. 0,01 - 20 Gew.-% an Spaltkatalysatoren zugesetzt enthalten, bevor sie einem erfindungsgemäßen Verfahren unterworfen wird.

Flüssigkeiten F, die Michael-Addukte enthalten, die bei der Herstellung von Acrylsäure gebildet wurden, fallen z. B. bei Verfahren zur Herstellung von Acrylsäure an, bei denen man ein durch katalytische Gasphasen-Partialoxidation einer C₃-Vorläuferverbindung der Acrylsäure erhaltenes, Acrylsäure enthaltendes, Produktgasgemisch, gegebenenfalls nach zuvor erfolgter Kühlung, in sich selbst aufsteigend unter Seitenabzug einer rohen Acrylsäure in einer mit trennwirksamen Einbauten versehenen Trennkolonne fraktionierend kondensiert, und die dabei gebildete, Michael-Addukte der Acrylsäure enthaltende Flüssigkeit aus dem Sumpf der Kondensationskolonne kontinuierlich herausführt und als Flüssigkeit F der Rückspaltung der darin enthaltenen Michael-Addukte der Acrylsäure zuführt (vgl. z. B. DE-A 10 2007 004 960, WO 2004/035514, DE-A 10 2006 062 258 und die Deutsche Anmeldung mit dem Aktenzeichen 10 2008 001 435.4).

In typischer Weise kann eine solche erfindungsgemäß zu behandelnde Flüssigkeit F, einschließlich gegebenenfalls vorab einer Durchführung des erfindungsgemäßen Verfahrens zugesetzter (Rück)Spaltkatalysatoren enthalten:

| | |
|---|---|
| 10 bis 50 Gew.-% | Acrylsäure-Michael-Oligomere (Michael-Addukte), |
| wenigstens 40 Gew.-% | radikalisches Acrylsäurepolymerisat, |
| bis zu 25 Gew.-% | monomere Acrylsäure, |
| bis zu 2 Gew.-% | Polymerisationsinhibitor, und |
| bis zu 15 Gew.-% | sonstige Verbindungen. |

Häufig enthalten solche Flüssigkeiten F auch:

| | |
|---|---|
| 10 bis 50 Gew.-% | Acrylsäure-Michael-Oligomere (Michael-Addukte), |
| 40 bis 80 Gew.-% | radikalisches Acrylsäurepolymerisat, |
| 5 bis 20 Gew.-% | monomere Acrylsäure, |
| 0,1 bis 2 Gew.-% | Polymerisationsinhibitor, und |
| 1 bis 15 Gew.-% | sonstige Verbindungen. |

Sie können aber auch enthalten:

| | |
|---|---|
| 10 bis 40 Gew.-% | Acrylsäure-Michael-Oligomere, (Michael-Addukte) |
| 50 bis 70 Gew.-% | radikalisches Acrylsäurepolymerisat, |
| 5 bis 15 Gew.-% | monomere Acrylsäure, |
| 0,1 bis 1 Gew.-% | Polymerisationsinhibitor, und |
| 1 bis 15 Gew.-% | sonstige Verbindungen; |

oder:

| | |
|---|---|
| 15 bis 35 Gew.-% | Acrylsäure-Michael-Oligomere, |
| 50 bis 70 Gew.-% | radikalisches Acrylsäurepolymerisat, |
| 5 bis 15 Gew.-% | monomere Acrylsäure, |
| 0,1 bis 1 Gew.-% | Polymerisationsinhibitor, und |
| 1 bis 15 Gew.-% | sonstige Verbindungen |

Häufig sind dabei in den Flüssigkeiten F 40 bis 60 Gew.-% der in ihnen enthaltenen Acrylsäure-Michael-Oligomere lediglich Acrylsäure-Michael-Dimere und 15 bis 30 Gew.-% Acrylsäuresäure-Michael-Trimere.

Selbstverständlich können dem erfindungsgemäßen Verfahren zu unterwerfende Flüssigkeiten F auch resultieren, wenn die im Produktgasgemisch der heterogen katalysierten Gasphasenpartialoxidation enthaltene Acrylsäure durch Absorption in ein Absorptionsmittel in die flüssige Phase überführt und die Acrylsäure nachfolgend mittels rektifikativer und/oder kristallisativer Trennverfahren aus dem Absorbat abgetrennt wird, wie es z. B. die DE-A 103 36 386 und die DE-A 29 01 783 offenbart.

In der Regel enthalten erfindungsgemäß zu behandelnde Flüssigkeiten F, bezogen auf ihr Gewicht, wenigstens 10 Gew.ppm, häufig wenigstens 50 Gew.ppm und vielfach wenigstens 150 Gew.ppm an Polymersationsinhibitor. In der Regel beträgt der Gehalt an Polymerisationsinhibitoren von Flüssigkeiten F in gleicher Weise bezogen ≤ 1 Gew.-%, oder ≤ 0,5 Gew.-%. Neben Phenothiazin und/oder Hydrochinonmonomethylether und deren Folgeprodukte kommen als solche Polymerisationsinhibitoren auch Verbindungen wie Alkylphenole (z. B. o-, m- oder p-Kresol (Methylphenol)), Hydroxyphenole (z. B. Hydrochinon), Tocopherole (z. B. o-Tocopherol) und N-Oxyle wie Hydroxy - 2,2,6,6-tetramethyl-piperidi-N-oxyl und die anderen in der Literatur bekannten Inhibitoren in Betracht.

Bei den von Acrylsäure, den Estern von Acrylsäure sowie den Michael-Addukten verschiedenen Bestandteilen von Flüssigkeiten F handelt es sich in erster Linie um bei Normaldruck höher als Acrylsäure und ihre Ester siedende Verbindungen.
Bei Bedarf können sie auch Tenside zugesetzt enthalten, wie es z. B. in der Deutschen Anmeldung mit dem Aktenzeichen 10 2008 001 435.4 beschrieben ist.

Als Trennkolonne K können für das erfindungsgemäße Verfahren grundsätzlich alle an sich bekannten Typen von Rektifikationskolonnen verwendet werden.

Dies sind alle trennwirksame Einbauten enthaltenden Kolonnen, wobei als trennwirksame Einbauten z. B. Packungen, Füllkörperschüttungen und/oder Böden in Betracht kommen. Die trennwirksamen Einbauten verfolgen den Zweck, die Austauschfläche zwischen in der Trennkolonne K aufsteigender Gasphase und in der Trennkolonne K ablaufender Flüssigkeit zu vergrößern und dadurch sowohl den Stoff- als auch den Wärmeaustausch zwischen beiden Phasen zu verbessern. Sie sind sowohl für in der Trennkolonne K aufsteigendes Gas als auch in der Trennkolonne K ablaufende Flüssigkeit durchlässig.

Erfindungsgemäß bevorzugt enthält die Trennkolonne K nur Böden und/oder Packungen. Als Böden werden dabei mit Vorteil Dual-Flow-Böden verwendet und mit besonderem Vorteil enthält die Trennkolonne K ausschließlich Dual-Flow-Böden als trennwirksame Einbauten.

Unter Dual-Flow-Böden werden in dieser Schrift Platten mit einfachen Durchtrittstellen (Löcher, Schlitze etc.) verstanden. Das in der Trennkolonne K aufsteigende Gas und die in der Trennkolonne absteigende Flüssigkeit treten entgegengesetzt strömend durch die gleichen Durchtrittstellen. Der Querschnitt der Durchtrittstellen wird in an sich bekannter Weise der Belastung der Trennkolonne K angepasst. Ist er zu klein, strömt das aufsteigende Spaltgas mit so hoher Geschwindigkeit durch die Durchtrittstellen, dass die in der Trennkolonne K absteigende Flüssigkeit im Wesentlichen ohne Trennwirkung mitgerissen wird. Ist der Querschnitt der Durchtrittstellen zu groß, bewegen sich aufsteigendes Spaltgas und absteigende Flüssigkeit im Wesentlichen ohne Austausch aneinander vorbei und der Boden läuft Gefahr, trocken zu laufen. Üblicherweise weisen Dual-Flow-Böden kein Ablaufrohr auf, das sie mit dem nächsten Boden verbindet. Selbstredend kann jeder Dual-Flow-Boden mit den Wänden der Rektifikationskolonne bündig abschließen. Er kann aber auch über Stege mit diesen verbunden sein. Mit abnehmender Belastung der Rektifikationskolonne laufen Dual-Flow-Böden im Unterschied zu hydraulisch abgedichteten Querstromböden trocken.

Die Durchtrittstellen der Dual-Flow-Böden sind bevorzugt kreisrunde Löcher mit einem innerhalb des Bodens einheitlichen Kreisdurchmesser. Letzterer beträgt zweckmäßig 10 bis 30 mm. Im oberen Teil der Kolonne beträgt er mit Vorteil 10 bis 20 mm. Bzw. 10 bis 15 mm, und im unteren Teil der Trennkolonne K beträgt er mit Vorteil 20 bis 30 mm. Die kreisrunden Löcher sind über einen individuellen Dual-Flow-Boden vorzugsweise in strenger Dreiecksteilung gleichmäßig angeordnet (vlg. DE-A 102 30 219). Ferner zeigt der Stanzgrat der in den Dual-Flow-Böden herausgestanzten Durchtrittsöffnungen in der Trennkolonne K bevorzugt nach unten. Üblicherweise sind die Dual-flow-Böden in der Trennkolonne K äquidistant angeordnet. In typischer Weise liegt der Bodenabstand bei 300 bis 500 mm. Günstig ist auch ein Bodenabstand von 400 mm.

Die Zuführstelle I, an der die Flüssigkeit F in die Trennkolonne K hineingeführt wird, befindet sich erfindungsgemäß oberhalb der untersten trennwirksamen Einbauten in der Trennkolonne K. Im Fall einer Bodenkolonne befindet sich die Zuführstelle I somit oberhalb des untersten Bodens.

Handelt es sich bei der Trennkolonne K um eine reine Packungskolonne, befindet sich die Zuführstelle I oberhalb der untersten Packung.

Eine reine Dual-Flow-Boden Trennkolonne K kann beim erfindungsgemäßen Verfahren bis zu 60 Dual-Flow-Böden oder mehr enthalten. Mit Vorteil weisen diese ein Öffnungsverhältnis (das Verhältnis D : U, gebildet aus dem Flächenanteil des Bodens, der für das Spaltgas durchlässig ist (D) und der Gesamtfläche des Bodens (U) von 10 bis 20 %, bevorzugt von 10 bis 15 % auf.

Erfindungsgemäß vorteilhaft befindet sich die Zuführstelle I im Fall einer reinen Dual-Flow-Bodenkolonne (z. B. mit ≥ 40 äquidistanten) Dual-Flow-Böden im Bereich des, von unten nach oben betrachtet, vierten bis zehnten Dual-Flow-Boden. Anwendungstechnisch zweckmäßig entspricht die Zuführtemperatur T^{Z} der Flüssigkeit F an der Zuführstelle I derjenigen Temperatur, die die in der Trennkolonne K absteigende Flüssigkeit an dieser Stelle aufweist. Mit Vorteil weichen die beiden vorgenannten Temperaturen um nicht mehr als 10 % (bezogen auf ihren arithmetischen Mittelwert) voneinander ab. Anwendungstechnisch zweckmäßig ist die Trennkolonne K ebenso wie ihre Zuführ- und Abfuhrleitungen gegen die Umgebung wärmeisoliert.

In der Regel sind Trennkolonnen K mit 2 bis 25 theoretischen Böden ausreichen. Als theoretischer Boden (oder theoretische Trennstufe) soll dabei diejenige Raumeinheit des trennwirksame Einbauten enthaltenden Trennraums der Trennkolonne K verstanden werden, die eine Stoffanreicherung entsprechend dem thermodynamischen Gleichgewicht ohne Energieverlust bewirkt.

Vorzugsweise befindet sich die Zuführstelle I einer erfindungsgemäß verwendeten Trennkolonne K im Bereich des, von unten nach oben betrachtet, zweiten bis achten theoretischen Bodens.

Die Erzeugung der Rücklaufflüssigkeit für die Trennkolonne K kann durch direkte und/oder indirekte Kühlung des in den (zum) Kopfraum der Trennkolonne K strömenden Gasstroms G erfolgen. Erfindungsgemäß vorteilhaft wird die Methode der direkten Kühlung angewendet.

Dazu wird in einfachster Weise der durch die oberste trennwirksame Einbaute der Trennkolonne K in den darüber befindlichen Kopfraum strömende Gasstrom G einer Quenchvorrichtung zugeführt, die z. B. in den Kopfraum integriert sein kann (in diesem Fall ist der Kopfraum vom Trennraum z. B. über einen Kaminboden abgetrennt; Sumpfraum und Kopfraum enthalten keine trennwirksamen Einbauten).

Grundsätzlich kann die Quenchvorrichtung aber auch aus der Trennkolonne K räumlich ausgelagert sein. Als eine solche Quenchvorrichtung können alle im Stand der Technik für diesen Zweck bekannten Vorrichtungen (z. B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden. Mit Vorteil wird eine Gleichstromvorrichtung (z. B. eine mit Prallplattendüse) verwendet. Zur indirekten Kühlung der Quenchflüssigkeit wird diese üblicherweise über einen (indirekten) Wärmeübertrager bzw. Wärmeaustauscher geführt. Diesbezüglich eignen sich alle gängigen Wärmeübertrager oder Wärmeaustauscher. Als bevorzugt seien Rohrbündelwärmeaustauscher, Plattenwärmeaustauscher und Luftkühler genannt. Geeignete Kühlmedien sind Luft beim entsprechenden Luftkühler und Kühlflüssigkeiten, insbesondere Wasser (z. B. Oberflächenwasser), bei den anderen Kühlvorrichtungen. Anwendungstechnisch zweckmäßig wird als Quenchflüssigkeit eine Teilmenge des beim Quenchen gebildeten Kondensats verwendet. Die andere Teilmenge des beim Quenchen gebildeten Kondensats wird normalerweise im wesentlichen als Rücklaufflüssigkeit auf die oberste trennwirksame Einbaute in der Trennkolonne K rückgeführt (bei Bedarf kann auch ein Teil des Kondensats ausgelassen werden). Selbstredend kann die Kondensation auch ausschließlich mit in den Kopfraum integrierten und/oder aus dem Kopfraum ausgelagerten indirekten Wärmeaustauschern durchgeführt werden, indem der Gasstrom G durch diese hindurchgeführt wird.

Anwendungstechnisch vorteilhaft wird die Trennkolonne K polymerisationsinhibiert betrieben. Als solche Polymerisationsinhibitoren können für diesen Zweck prinzipiell alle im Stand der Technik für Acrylmonomere bekannten Polymerisationsinhibitoren eingesetzt werden. Beispielhaft genannt seien als solche Phenothiazin (PTZ) und p-Methoxyphenol (MEHQ).

Häufig werden diese beiden kombiniert angewendet. Zweckmäßigerweise werden sie der Rücklaufflüssigkeit in reinem Rückspaltprodukt gelöst zugesetzt. MEHQ wird vorzugsweise als Schmelze zudosiert.

Die Zuführstelle I (darunter wird die Stelle im Sumpfraum der Trennkolonne K verstanden, an der der Teilstrom II aus der Zulaufleitung in den Sumpfraum austritt) befindet sich beim erfindungsgemäßen Verfahren unterhalb der untersten trennwirksamen Einbaute der Trennkolonne K und oberhalb des Stands S der Sumpfflüssigkeit (der in den Sumpfraum der Trennkolonne K ablaufenden Flüssigkeit). Erfindungsgemäß vorteilhaft ist der Stand S der (der Sumpfflüssigkeit) in den Sumpfraum ablaufenden Flüssigkeit so eingestellt, dass er weniger als 40 %, vorzugsweise weniger als 30 % und besonders bevorzugt weniger als 20 % des Abstands A beträgt. In der Regel wird der Stand S jedoch nicht weniger als 5 % des Abstands A betragen (Sicherheitsflüssigkeitsstand).

Erfindungsgemäß vorteilhaft wird diese Sicherheitshöhe mit geringem Sumpfflüssigkeitsvolumen realisiert, in dem man im Sumpfraum Verdrängungskörper anbringt oder den Sumpfraum zu seinem unteren Ende hin einzieht (vgl. Fig. 6 der DE-A 103 32 758 oder auch die EP-A 1 095 685 sowie Figur 1 der DE-A 10 2004 015 727).

Mit besonderem Vorteil ist der Sumpfraum zu seinem unteren Ende hin eingezogen und der Stand S der in den Sumpfraum ablaufenden Flüssigkeit (der Stand der Sumpfflüssigkeit) befindet sich in dem Abschnitt des Sumpfraums, in dem der Sumpfraum eingezogen ist (d. h., in dem Abschnitt, in welchem er einen verkleinerten Innendurchmesser aufweist).

In der Regel befindet sich die Zuführstelle II beim erfindungsgemäßen Verfahren wenigstens 0,25 • A oberhalb des Stands S der Sumpfflüssigkeit (oberhalb des Flüssigkeitsspiegels (Pegels) der Sumpfflüssigkeit).

Erfindungsgemäß erfolgt die Rückführung des Teilstroms II in den Sumpfraum der Trennkolonne K so, dass der Teilstrom II im Sumpfraum der Trennkolonne K nicht auf die Sumpfflüssigkeit gerichtet ist (d. h., die Verlängerung des Strömungsvektors derjenigen Strömung mit der der Teilstrom II aus der entsprechenden Zuleitung in den Sumpfraum austritt, trifft nicht auf die Sumpfflüssigkeit, sondern auf einen von der Sumpfflüssigkeit verschiedenen materiellen Gegenstand (z. B. die Wand des Sumpfraums, eine Prallplatte etc.).

In einfacher Weise ist die vorgenannte erfindungsgemäße Bedingung dadurch zu verwirklichen, dass der Teilstrom I horizontal in den Sumpfraum hineinströmt (z. B. über einen einfachen Zulaufstutzen).

Mit Vorteil erfolgt der Zustrom des Teilstroms II in den Sumpfraum der Trennkolonne K jedoch aus einer in den Sumpfraum hineingeführten Leitung A, deren Austrittsöffnung im Sumpfraum zwar nach unten zeigt, jedoch nicht auf die Sumpfflüssigkeit, sondern auf eine Prallvorrichtung A gerichtet (auf einen Strömungsverteiler gerichtet) ist, die (der) im Sumpfraum oberhalb des Stands S der Sumpfflüssigkeit angebracht ist, und die den Teilstrom II bei seinem Aufprall auf der Prallvorrichtung nach oben gerichtet umlenkt (vgl. z. B. Figur 1 der DE-A 10 2004 015 727).

Wird in der Trennkolonne K als Schleppmittel (Schleppgas oder auch Unterstützgas) für die Rückspaltprodukte (Spaltprodukte) "Strippgas" mitverwendet, so wird dieses beim erfindungsgemäßen Verfahren ebenfalls oberhalb des Stands S der Sumpfflüssigkeit und unterhalb der untersten trennwirksamen Einbauten der Trennkolonne K in den Sumpfraum der Trennkolonne K geführt (und strömt von dort aus in den Kopfraum der Trennkolonne K). Letzteres wiederum so, dass der Gasstrom im Sumpfraum der Trennkolonne K nicht auf die Sumpfflüssigkeit gerichtet ist (d. h., die Verlängerung des Strömungsvektors, mit der der Gasstrom aus der entsprechenden Zuleitung in den Sumpfraum austritt, trifft nicht auf die Sumpfflüssigkeit).

In einfacher Weise ist dies dadurch zu verwirklichen, dass der Strippgasstrom horizontal in den Sumpfraum hineinströmt (z. B. über einen einfachen Zulaufstutzen). Die Zuführstelle dafür kann sich sowohl oberhalb als auch unterhalb der Zuführstelle II befinden.

Mit Vorteil erfolgt der Zustrom eines Strippgasstroms in den Sumpfraum der Trennkolonne K jedoch aus einer in den Sumpfraum hineingeführten Leitung B, deren Austrittsöffnung im Sumpfraum nach unten zeigt, jedoch nicht auf die Sumpfflüssigkeit sondern auf eine Prallvorrichtung B gerichtet (auf einen Strömungsverteiler gerichtet) ist, die (der) im Sumpfraum oberhalb des Stands S der Sumpfflüssigkeit angebracht ist, und die den Strippgasstrom bei seinem Aufprall auf der Prallvorrichtung nach oben gerichtet umlenkt (vgl. z. B. Figur 1 der DE-A 10 2004 015 727).

Anwendungstechnisch zweckmäßig wird der Teilstrom II über eine mit ihrer Austrittsöffnung A auf eine Prallvorrichtung A gerichtete Leitung A und simultan dazu ein Strippgasstrom über eine mit ihrer Austrittsöffnung B auf eine Prallvorrichtung B gerichtete Leitung B in den Sumpfraum der Trennkolonne K hineingeführt. Dabei können sich die Prallvorrichtung A und mit dieser auch die Austrittsöffnung A sowohl oberhalb als auch unterhalb der Prallvorrichtung B und der zu ihr gehörigen Austrittsöffnung B befinden. Anwendungstechnisch bevorzugt umhüllt die Leitung A die Leitung B (beide Leitungen bilden (vorzugsweise gegeneinander thermisch isoliert) ein "koaxiales Doppelrohr") und die Austrittsöffnungen A und B befinden sich im wesentlichen auf derselben Höhe und die Prallvorrichtung B ist mit der Prallvorrichtung A identisch.

Aus Gründen der Polymerisationsinhibierung enthält das Strippgas vorzugsweise molekularen Sauerstoff. Als solches kommt z. B. Luft, an Sauerstoff entreicherte Luft und/oder Kreisgas in Betracht. Unter Kreisgas wird dabei das Restgas verstanden, das verbleibt, wenn man aus dem Produktgasgemisch der zur Herstellung von Acrylsäure angewandten heterogen katalysierten Gasphasenpartialoxidation einer C₃-Vorläuferverbindung (z. B. Propen, Propan, Acrolein, Glycerin) die Acrylsäure durch Absorption mit einem flüssigen Absorptionsmittel oder durch fraktionierende Kondensation in den flüssigen Aggregatzustand überführt (vgl. z. B. WO 2004/035514, DE-A 103 32 758, DE-A 10 2007 004 960). Die überwiegende Menge dieses Restgases wird im Kreis in die Partialoxidation rückgeführt, um das Reaktionsgasgemisch zu verdünnen.

In der Regel wird aus dem vorgenannten Restgas vorab seiner Verwendung als Strippgas noch eine wässrige Phase auskondensiert, die in der Regel Restmengen an Acrylsäure enthält (Sauerwasser), die aus dieser wässrigen Phase durch Extraktion mit einem organischen Extraktionsmittel in das resultierende Extrakt abgetrennt werden kann. Vorab einer Verwendung des Restgases als Strippgas im erfindungsgemäßen Verfahren, kann das Restgas auch noch zum Freistrippen der Acrylsäure aus vorgenanntem Extrakt eingesetzt worden sein (vgl. DE-A 10 2007 004 960). Normalerweise wird das Strippgas mit einer Temperatur zugeführt, die unterhalb von T^{SU} und oberhalb von 100 °C, teilweise oberhalb von 150 °C, liegt.

Bezogen auf 1 kg von an der Zuführstelle I je Stunde zugeführte Flüssigkeit F, kann der zugeführte Strippgasstrom z. B. 1 bis 100 kg/h betragen. Strippgas wird insbesondere dann mitverwendet, wenn der Umlaufwärmeaustauscher UW ein Zwangsumlaufentspannungswärmeaustauscher ist.

Durch die Zudosierung von Strippgas kann der Partialdruck der (Rück)Spaltprodukte in der Trennkolonne K in entsprechender Weise abgesenkt werden, wie durch das Aufprägen (Anlegen) von vermindertem Druck (Unterdruck).

Wird beim erfindungsgemäßen Verfahren der Trennkolonne K kein Strippgas zugeführt wird am Kolonnenkopf ein Arbeitsdruck angewendet, der vorteilhaft unterhalb von 1 bar liegt (und z. B. 100 mbar beträgt).

Wird ein Strippgas mitverwendet, liegt der Arbeitsdruck am Kopf der Trennkolonne K in der Regel bei einem Druck von > 1 bis 3 bar, bevorzugt 1,5 bis 2,5 bar.

Die Temperatur T^{SU} der im Sumpfraum mit dem Stand S befindlichen Sumpfflüssigkeit liegt in der Regel im Bereich von 130 bis 250 °C, häufig 150 bis 190 °C und vielfach 160 bis 180 °C.

Die Differenz T^{RS} - T^{SU} wird beim erfindungsgemäßen Verfahren in der Regel wenigsten 2 °C, vorzugsweise wenigstens 5 °C oder wenigstens 10 °C betragen. Normalerweise wird vorgenannte Temperaturdifferenz jedoch ≤ 100 °C, häufig sogar ≤ 80 °C und oft ≤ 50 °C betragen. Relativ zueinander sollten beide Temperaturen beim erfindungsgemäßen so gewählt werden, dass die Rückspaltrate im Spaltreaktor größer ist, als die Schwersiederbildungsrate im Sumpf der Trennkolonne K.

Bei einem erfindungsgemäß einzusetzenden indirekten Umlaufwärmeaustauscher UW erfolgt die Wärmeübertragung nicht im durch Mischen erzwungenen direkten Kontakt zwischen fluidem Wärmeträger und zu erwärmendem flüssigem Gemisch. Vielmehr erfolgt die Wärmeübertragung indirekt zwischen den durch eine Trennwand getrennten Fluiden. Die für den Wärmetransport aktive Trennfläche des Wärmeüberträgers (Wärmeaustauschers) wird als Wärmeaustausch- oder Übertragungsfläche bezeichnet, und der Wärmetransport folgt den bekannten Gesetzen des Wärmedurchgangs.

Erfindungsgemäß wesentlich ist, dass beim erfindungsgemäßen Verfahren der indirekte Umlaufwärmeaustauscher UW sowohl vom fluiden Wärmeträger als auch von der Flüssigkeit F durchströmt wird. D. h., beide strömen in den Wärmeaustauscher hinein und anschließend wieder heraus (der eine durchströmt den wenigstens einen Primärraum und der andere den wenigstens einen Sekundärraum).

Als fluide Wärmeträger kommen für das erfindungsgemäße Verfahren grundsätzlich alle möglichen heißen Gase, Dämpfe und Flüssigkeiten in Betracht.

In erster Linie zählt dazu Wasserdampf, der sich auf unterschiedlichen Drücken und Temperaturen befinden kann. Häufig ist es günstig, wenn der Wasserdampf beim Durchströmen des indirekten Wärmeaustauschers kondensiert (Sattdampf).

Alternativ kommen als fluide Wärmeträger Öle, Schmelzen, organische Flüssigkeiten sowie heiße Gase in Betracht. Beispiele dafür sind Siliconverbindungen wie Tetraarylsilikat, Diphenyl enthaltendes Gemisch aus 74 Gew.-% Diphenylether und 26 Gew.-% Diphenyl, das Azeotrop aus Diphenyl und Diphenylether, chloriertes nichtbrennendes Diphenyl sowie Mineralöle und Druckwasser.

Der Unterschied (T^{W} - T^{SU}) zwischen derjenigen Temperatur T^{W}, mit der der fluide Wärmeträger bei der Durchführung des erfindungsgemäßen Verfahrens in den wenigstens einen Primärraum des Umlaufwärmeaustauschers UW eintritt und derjenigen Temperatur T^{SU}, mit der der wenigstens eine Teilstrom I im Wesentlichen in den wenigstens einen Sekundärraum desselben Umlaufwärmeaustauschers UW eintritt, kann z. B. 1 bis 150 °C, häufig 5 bis 100 °C, oder 10 bis 80 °C, vielfach 20 bis 60 °C, oder vorzugsweise 15 bis 35 °C betragen.

Für das erfindungsgemäße Verfahren geeignete indirekte Umlaufwärmeaustauscher sind insbesondere Doppelrohr-, Rohrbündel-, Rippenrohr-, Spiral- oder Plattenwärmeüberträger. Doppelrohrwärmeüberträger bestehen aus zwei ineinanderliegenden Rohren.

Mehrere dieser Doppelrohre können zu Rohrwänden verbunden werden. Das Innenrohr kann glatt oder zum Verbessern des Wärmeübergangs mit Rippen versehen sein. Auch kann in Einzelfällen ein Rohrbündel das Innenrohr vertreten. Die im Wärmeaustausch stehenden Fluide können sich im Gleichstrom oder im Gegenstrom bewegen. Die Flüssigkeit F wird erfindungsgemäß zweckmäßig im Innenrohr aufwärts gefördert und heißer Wasserdampf strömt z. B. im Ringraum abwärts.

Besonders geeignet für das erfindungsgemäße Verfahren sind Rohrbündelwärmeüberträger als Umlaufwärmeaustauscher UW. Sie bestehen normalerweise aus einem abgeschlossenen weiten Mantelrohr, das die an Rohrböden befestigten zahlreichen glatten oder gerippten Übertragerrohre kleinen Durchmessers umschließt.

Der Abstand von Rohrmitte zu Rohrmitte der Bündelrohre beträgt anwendungstechnisch zweckmäßig das 1,3- bis 2,5-fache der Rohraußendurchmesser. Die entstehende große spezifische Wärmeaustauschfläche - als Austauschfläche je Einheit des Raumbedarfs - ist ein Vorzug des Rohrbündelwärmeübertragers. Die senkrecht oder waagrecht angeordneten Rohrbündelwärmeübertrager unterscheiden sich u. a. in der Rohrführung. Die Übertragerrohre können gerade, U-förmig gebogen oder auch als mehrgängiges Spiralrohrbündel ausgeführt sein.

Der erfindungsgemäß zu erwärmende wenigstens eine Teilstrom I strömt erfindungsgemäß bevorzugt innerhalb der Übertragerrohre (grundsätzlich kann er aber auch im die Übertragerrohre umgebenden Raum und der Wärmeträger in den Übertragerrohren strömen). Der fluide Wärmeträger (vorzugsweise Wassersattdampf) strömt erfindungsgemäß zweckmäßig außerhalb der Übertragerrohre. Leitbleche zur besseren Führung des fluiden Wärmeträgers im Mantelraum sind erfindungsgemäß zweckmäßig und dienen in der Regel dem Zusatzzweck, die Übertragerrohre zu stützen. Die Leitbleche erhöhen in der Regel die Strömungsgeschwindigkeiten im Mantelraum und u. a. damit die Wärmeübergangszahlen. Die Strömung verläuft im Mantelraum mit Vorteil quer zu den Übertragerrohren. Nach der Strömungsrichtung des Mantelraumfluids in Bezug auf die Übertragerrohre sind z. B. Längsstrom- und Kreuzstrom- sowie Querstromrohrbündelwärmeübertrager unterscheidbar. Grundsätzlich kann der fluide Wärmeübertrager auch mäanderförmig um die Übertragerrohre bewegt und lediglich über den Rohrbündelwärmeaustauscher betrachtet im Gleich- oder Gegenstrom zum erfindungsgemäß zu erwärmenden flüssigen Gemisch geführt werden. Auch Spiralrohrbündel-Wärmeübertrager nutzen in der Regel die Vorteile des Kreuzstroms. Die Rohre wechseln - von Lage zu Lage - rechts-linksgängig ab. Das Mantelraumfluid fließt im Gegenstrom zum Rohrfluid und umströmt die Spiralrohre im Kreuzstrom.

Im Einstromrohrbündelwärmeübertrager bewegt sich der erfindungsgemäß zu erwärmende wenigstens eine Teilstrom I durch alle Übertragerrohre in der gleichen Richtung.

Mehrstromrohrbündelwärmeübertrager enthalten in einzelne Sektionen unterteilte Rohrbündel (in der Regel enthalten die einzelnen Sektionen eine identische Anzahl von Rohren). Trennwände teilen sich an die Rohrböden (durch die die Übertragerrohre abgedichtet geführt und an denen sie befestigt sind) anschließende Kammern in Abschnitte auf und lenken den aus einer Sektion in den Kammerteil eintretenden wenigstens einen Teilstrom I in eine zweite Sektion um und damit zurück. Der erfindungsgemäß zu erwärmende wenigstens eine Teilstrom I durchfließt je nach Anzahl der Sektionen die Länge des Rohrbündelwärmeübertragers mehrmals (zweimal, dreimal, viermal etc.) mit hoher Geschwindigkeit in alternierender Richtung (Zweistrom-, Dreistrom-, Vierstrom- etc. -rohrbündelwärmeübertrager). Wärmeübergangszahl und Austauschweg nehmen entsprechend zu.

Plattenwärmeübertrager (Plattenwärmeaustauscher) sind normalerweise nach Art der Filterpressen in der Regel aus gewellten oder anderweitig profilierten, mit Kanälen für den fluiden Wärmeträger und das aufzuwärmende flüssige Gemisch versehenen Platten (in der Regel aus Graphit oder Metall, z. B. Edelstahl) in kompakter Bauweise zusammengesetzt. Die beiden Wärme austauschenden Fluide fließen dann im Gleich-, Gegen- und/oder Querstrom als dünne Schichten wechselnd (z. B. auf- und abwärts) durch ihre Kammerreihen und stehen an beiden Kammerwänden miteinander in Wärmeübertragung. Die gewellten Plattenprofile erhöhen die Turbulenz und verbessern die Wärmeübergangszahlen. Für den erfindungsgemäßen Zweck geeignete Plattenwärmeaustauscher sind z. B. beschrieben in der EP-A 107 9194, der US-A 6,382,313, der EP-A 123 2004 und der WO 01/32301. Rohrbündelwärmeaustauscher sind z.B. beschrieben in der EP-A 700 893, EP-A 700 714 und DE-A 443 1949. Spiral- und Rippenrohrwärmeaustauscher findet man z. B. beschrieben in Vauck/Müller, Grundoperationen chemischer Verfahrenstechnik, 4. Auflage, Verlag Theodor Steinkopf, Dresden (1974) sowie in Ullmanns Encyclopädie der technischen Chemie, Band 2, Verfahrenstechnik I (Grundoperationen), 4. Auflage, 1972, S. 432 ff.

Für das erfindungsgemäße Verfahren wesentlich ist, dass der wenigstens eine Teilstrom I durch den wenigstens einen Sekundärraum des indirekten Umlaufwärmeaustauschers UW, mit Hilfe der Pumpe P, zwangsgefördert wird. Erfindungsgemäß bevorzugt werden zur Durchführung des erfindungsgemäßen Verfahrens daher als Umlaufwärmeaustauscher UW Zwangsumlaufröhrenwärmeaustauscher (Zwangsumlaufrohrbündelwärmeübertrager) eingesetzt.
Vorzugsweise wird der wenigstens eine Teilstrom I dabei in den Röhren derselben zwangsgefördert.

Beispielsweise kann zur Durchführung des erfindungsgemäßen Verfahrens ein Dreistromrohrbündelwärmeübertrager verwendet werden, durch dessen Rohre der wenigstens eine Teilstrom I zwangsgefördert wird.

D. h., die Rohrinnenräume bilden die Sekundärräume des Wärmeübertragers. Die Außenrohrdurchmesser können 38 mm betragen, bei einer Wanddicke der Rohre von 2 mm. Bei einer Länge der Rohre von 4800 mm beträgt deren Gesamtzahl anwendungstechnisch zweckmäßig 234 (jeweils 78 Rohre für eine Strömungsrichtung). Die Rohrteilung beträgt gleichzeitig vorteilhaft 48 mm (30°-Teilung). Durch 9 zwischen den Rohrböden (in denen die Austauscherrohre befestigt sind) angebrachte Umlenkscheiben (Scheibendicke: jeweils 5 mm) wird der die Wärmeübertragerrohre umgebende zylindrische Raum (Primärraum) in 10 Längsabschnitte (Segmente) unterteilt. Alle 9 Umlenkscheiben sind im Prinzip kreisförmig. Der Kreisdurchmesser beträgt 859 mm. An jeder der kreisförmigen Umlenkscheiben ist jedoch ein halbmondförmiges Kreissegment abgeschnitten, dessen Fläche 35,8 % der Gesamtfläche beträgt, so dass ein entsprechender Durchtritt für Wasserdampf als Wärmeträger entsteht, wobei diese Durchtritte aufeinanderfolgend alternierend einander gegenüberliegend angebracht sind (im übrigen sind die Umlenkbleche an der Behälterwand abdichtend befestigt; dort wo die Wärmeübertragerrohre auf die Umlenkbleche stoßen, sind entsprechende Bohrungen in den Umlenkblechen). Durch den die Wärmeübertragerrohre umgebenden Raum wird als Wärmeträger anwendungstechnisch zweckmäßig Wasserdampf geführt. Der Eintritt von Wasserdampf und dem wenigstens einen Teilstrom I in den Dreistromrohrbündelwärmeübertrager befindet sich anwendungstechnisch günstig auf derselben Seite des Wärmeübertragers.
Alternativ kann für das erfindungsgemäße Verfahren auch ein Dreizehnstromrohrbündelwärmeübertrager eingesetzt werden, durch dessen Rohre der wenigstens eine Teilstrom I zwangsgefördert wird (so wie nachfolgend beschrieben im weiteren Verlauf dieser Schrift als Dreizehnstromrohrbündelwärmeübertrager D* bezeichnet). Erfindungsgemäß vorteilhaft ist der Zylinder, der den Primärraum umschließt, dabei mit einem Kompensator (Abmaße Kompensator: Durchmesser = 2,075 m; Höhe = 670 mm; 3 Faltenbalge; Einbauort auf halber Höhe des senkrecht ausgerichteten Primärraums) ausgestattet, der die spannungsarme Wärmeausdehnung des Apparates beim Aufheizen und Abkühlen ermöglicht.

Die Außenrohrdurchmesser können wiederum 38 mm betragen, bei einer Wanddicke der Rohre von 2 mm. Bei einer Länge der Rohre von 5000 mm beträgt deren Gesamtzahl anwendungstechnisch zweckmäßig 1066 (jeweils 82 Rohre für eine Strömungsrichtung). Die Rohrteilung beträgt gleichzeitig vorteilhaft 47 mm (60°-Teilung). Durch 9 zwischen den Rohrböden (in denen die Austauscherrohre befestigt sind) angebrachte Umlenkscheiben (Scheibendicke: jeweils 10 mm) wird der die Wärmeübertragerrohre umgebende zylindrische Raum (Primärraum) in 9 Längsabschnitte (Segmente) unterteilt. Alle 9 Umlenkscheiben sind im Prinzip kreisförmig. Der Kreisdurchmesser beträgt 1734 mm. An jeder der kreisförmigen Umlenkscheiben ist jedoch ein halbmondförmiges Kreissegment abgeschnitten, dessen Fläche 15 % der Gesamtfläche beträgt, so dass ein entsprechender Durchtritt für Wasserdampf als Wärmeträger entsteht, wobei diese Durchtritte aufeinanderfolgend alternierend einander gegenüberliegend angebracht sind (im übrigen sind die Umlenkbleche an der Behälterwand abdichtend befestigt; dort wo die Wärmeübertragerrohre auf die Umlenkbleche stoßen, sind entsprechende Bohrungen in den Umlenkblechen). Durch den die Wärmeübertragerrohre umgebenden Raum wird als Wärmeträger anwendungstechnisch zweckmäßig Wasserdampf geführt. Der Eintritt von Wasserdampf und dem wenigstens einen Teilstrom I in den Dreizehnstromrohrbündelwärmeübertrager befindet sich anwendungstechnisch günstig auf derselben Seite des Wärmeübertragers.

Der Arbeitsdruck auf der Druckseite der Pumpe P (vorab Eintritt des wenigstens einen Teilstroms I in den wenigstens einen Sekundärraum des Umlaufwärmeaustauschers UW) beträgt beim erfindungsgemäßen Verfahren häufig 4 bis 6 bar.

Im Übrigen kann z. B. wie in der DE-AS 12 79 015, der EP-A 780 360, der DE-A 197 01 737, der DE-A 23 39 519, der DE-A 29 01 783, der DE-A 103 32 758, der Deutschen Anmeldung mit dem Aktenzeichen 10 2008 001 435.4, der DE-A 10 2006 062 258, der DE-A 10 2007 004 960 sowie der WO 2004/035514 beschrieben, verfahren werden.

Häufig ist bei erfindungsgemäßen Verfahren der Zwangsumlaufwärmeaustauscher auch als ein Zwangsumlaufentspannungswärmeübertrager, vorzugsweise ein Zwangsumlaufrohrbündelentspannungswärmeübertrager ausgebildet. Dieser ist im Unterschied zum Fall eines reinen Zwangsumlaufwärmeübertragers von der Zuführstelle I in der Trennkolonne K normalerweise durch eine Drosselvorrichtung (z. B. im einfachsten Fall durch eine Lochblende (oder sonstige Blende); alternativ kommt auch ein Ventil in Betracht) getrennt.

Durch vorstehende Maßnahme wird ein Sieden des umgepumpten wenigstens einen Teilstroms I innerhalb des wenigstens einen Sekundärraums des Wärmeübertragers ((Wärmeaustauschers) - z. B. in den Rohren des Rohrbündelwärmeübertragers) unterdrückt. Der umgepumpte wenigstens eine Teilstrom I wird innerhalb des wenigstens einen Sekundärraums bezüglich des im Sumpfraum der Trennkolonne K herrschenden Gasdrucks GD vielmehr überhitzt und der Siedprozess so vollständig auf die Durchtrittseite der Drosselvorrichtung verlagert (d. h., der Inhalt der Rohre des Rohrbündelwärmeübertragers liegt einphasig vor, der Rohrbündelwärmeübertrager fungiert lediglich als Überhitzer). Die Drosselvorrichtung trennt den Wärmeübertrager (Wärmeaustauscher; z. B. Rohrbündelwärmeaustauscher) und die Zuführstelle II druckseitig und ermöglicht durch geeignet Wahl der Leistung der erfindungsgemäßen Pumpe die Einstellung eines oberhalb des im Sumpfraum herrschenden Gasdrucks GD liegenden Drosselvordrucks, der oberhalb des zur Temperatur T^{RS} gehörigen Siededrucks des aus dem wenigstens einen Sekundärraum des Wärmeübertragers ausströmenden Stoffstroms M * liegt. Die Siedeverdampfung findet in Strömungsvorrichtung erst hinter der Drossel statt. Die Anwendung von Zwangsumlaufentspannungswärmeaustauschern UW ist beim erfindungsgemäßen Verfahren bevorzugt.

Der Unterschied zwischen dem Drosselvordruck und dem im Sumpfraum vorliegenden Gasdruck GD beträgt dabei typisch 0,1 bis 5 bar, häufig 0,2 bis 4 bar und vielfach 1 bis 3 bar.

Die Temperatur des aus dem wenigstens einen Sekundärraum des Zwangsumlaufentspannungswärmeaustauschers UW herausströmenden Stoffstroms M * liegt beim Verlassen des wenigstens einen Sekundärraums (noch vor der Drossel) in der Regel wenigstens 5 °C oberhalb von T^{SU}.

Von wenigstens einem der beiden Ströme *M , M* * wird beim erfindungsgemäßen Verfahren ein Teilstrom als Reststrom ausgeschleust und seiner Entsorgung, z. B. seiner Verbrennung, zugeführt. Bezogen auf den Strom M beträgt der Reststrom in der Regel ≤ 1 Gew.-%.

Erfindungsgemäß bevorzugt wird lediglich ein Teilstrom des Stromes *M* zum Zweck seiner Entsorgung ausgelassen, und zwar bevor der dann verbleibende Teilstrom I des Stromes *Ṁ* in den wenigstens einen Sekundärraum des Umlaufwärmeaustauschers UW eintritt. Durch Zusatz eines organischen Lösungsmittels (z. B. Methanol) wird dieser zu entsorgende Teilstrom fluid gehalten.

Der Unterschied zwischen dem als Bestandteil der Flüssigkeit F der Rückspaltvorrichtung zugeführten Massenstrom an Michael-Addukten (*ṁ*ₑᵢₙ) und dem als Bestandteil des vorgenannten Reststroms ausgeschleusten Massenstrom an Michael-Addukten (*ṁ*ₐᵤₛ) ist beim erfindungsgemäßen Verfahren ein Maß für den Wirkungsgrad des Rückspaltverfahrens.

In der Regel beträgt [(*ṁ*ₑᵢₙ - *ṁ* ₐᵤₛ)/*ṁ*ₑᵢₙ] × 100 % (= Wirkungsgrad Q des Rückspaltverfahrens) beim erfindungsgemäßen Verfahren wenigstens 20 %, vorzugsweise wenigstens 30 % oder wenigstens 40 %. Vielfach beträgt Q beim erfindungsgemäßen Verfahren wenigstens 50 %. In günstigen Fällen (bei großer Verweilzeit) kann Q nahezu 100 % betragen. In der Regel beträgt Q beim erfindungsgemäßen Verfahren jedoch < 90 %. Bei Bedarf kann die Entnahme des Mengenstroms *Ṁ* (sein Ansaugen durch die Pumpe P) aus dem Sumpfraum der Trennkolonne K durch einen Trombenbrecher hindurch erfolgen.

Der Erfolg der erfindungsgemäßen Verfahrensweise wird darauf zurückgeführt, dass es in der Sumpfflüssigkeit (auf deren Weg zur Pumpe <p) in geringem Umfang noch zu Rückspaltungen von in der Sumpfflüssigkeit enthaltenen Michael-Addukten kommt. Liegt die Temperatur der Sumpfflüssigkeit oberhalb des Siedepunktes des am leichtesten siedenden Rückspaltproduktes, bildet selbiges (trotz in der Sumpfflüssigkeit nach unten zunehmenden hydrostatischen Druck) kleinste Gasbläschen, deren Auftrieb in der Sumpfflüssigkeit wenigstens teilweise nicht ausreichend ist, um sich der Saugwirkung der Pumpe P zu entziehen (ohne eine solche fortgesetzte Rückspaltung wäre die von der Pumpe P angesaugte Sumpfflüssigkeit im wesentlichen an Gasblasen frei (vgl. z. B. DE-A 103 32 758, Seite 9, Absatz [0082])). Über eine längere Betriebsdauer des erfindungsgemäßen Verfahrens sammeln sich diese Gasbläschen durch die Fliehkräfte im Zentrum eines geschlossenen radialen Laufrads einer Radialkreiselpumpe an (in einem geschlossenen radialen Laufrad können Gasbläschen nur schlecht mitgefördert werden) und verstopfen das Laufrad zunehmend ("Trombenbildung"), was schließlich das Zusammenbrechen der Saugleistung der Pumpe unter das erforderliche Mindestmaß zur Folge hat. Im Unterschied dazu lassen sich vorgenannte Gasbläschen von einer Radialkreiselpumpe mit halboffenem Laufrad offensichtlich vergleichsweise effektiv mitfördern.

Bei gleichen prozesstechnischen Bedingungen (Temperatur, Massendichte und Viskosität des geförderten Mediums, gleiches Pumpengehäuse, gleicher Antriebsmotor, gleicher Laufradradius und gleiche Förderschaufelgestaltung) ist die Förderleistung einer Radialkreiselpumpe mit halboffenem Laufrad zwar geringer wie bei einer Radialkreiselpumpe mit geschlossenem Laufrad, doch kann dies bei Bedarf durch eine Vergrößerung des Laufraddurchmessers kompensiert werden. In jedem Fall gewährleistet die Verwendung einer Radialkreiselpumpe mit halboffenem Laufrad beim erfindungsgemäßen Verfahren eine wesentlich zuverlässigere Flüssigkeitsförderung.

Der beim erfindungsgemäßen Verfahren bei der partiellen Kondensation des Gasstroms G verbleibende und ausgelassene Gasstrom kann ebenso wie eine gegebenenfalls nicht als Rücklaufflüssigkeit verwendete Teilmenge des dabei gebildeten Kondensats in gleicher Weise weiterverwendet werden, wie dies im Stand der Technik (z. B. DE-A 103 32 758, WO 2004/035514, WO 2008/090190, WO 2008/077767, EP-A 780 360, DE-A 197 01 737 und EP-A 1 357 105) bereits beschrieben ist.

Selbstverständlich können der Sumpfflüssigkeit der Trennkolonne K Dispergiermittel (z. B. Tenside) und/oder Entschäumer zugesetzt werden, wie sie z. B. in der deutschen Anmeldung Nr. 10 2008 001 435.4 emfohlen werden. Ihre Zugabe kann auch am Kopf der Trennkolonne K vorgenommen werden.

Damit umfasst die vorliegende Erfindung insbesondere die nachfolgenden Ausführungsformen:
1. Verfahren zur Rückspaltung von in einer Flüssigkeit F mit einem Gewichtsanteil, bezogen auf das Gewicht der Flüssigkeit F, von ≥ 10 Gew.-% enthaltenen Michael-Addukten, die bei der Herstellung von Acrylsäure oder deren Ester gebildet wurden, in einer Rückspaltvorrichtung, die wenigstens eine Pumpe P, eine Trennkolonne K, die von unten nach oben aus einem Sumpfraum, einem sich an den Sumpfraum anschließenden, trennwirksame Einbauten enthaltenden, Trennraum und einem sich an diesen anschließenden Kopfraum besteht und in der der Druck in der Gasphase von unten nach oben abnimmt, sowie einen indirekten Umlaufwärmeaustauscher UW, der wenigstens einen Sekundärraum und wenigstens einen, von dem wenigstens einen Sekundärraum durch eine materielle Trennwand D getrennten, Primärraum aufweist, umfasst, bei dem man kontinuierlich die Flüssigkeit F mit der Zuführtemperatur T^{Z} an einer Zuführstelle I, die sich oberhalb der untersten trennwirksamen Einbaute in der Trennkolonne K befindet, in die Trennkolonne K hineinführt, und an der tiefstgelegenen Stelle des Sumpfraums der Trennkolonne K mit der Pumpe P kontinuierlich einen Mengenstrom *Ṁ* der durch die trennwirksamen Einbauten in den Sumpfraum ablaufenden, Michael-Addukte enthaltenden, Flüssigkeit mit einer Temperatur T^{SU} entnimmt, so dass sich im Sumpfraum als Sumpfflüssigkeit ein Stand S der in diesen ablaufenden Flüssigkeit einstellt, der weniger als der halbe Abstand A, gemessen vom tiefstgelegenen Punkt der Trennkolonne K bis zur Unterseite der untersten trennwirksamen Einbaute in der Tennkolonne K, beträgt, während im über diesem Flüssigkeitsstand liegenden restlichen Raum des Sumpfraums ein Gasdruck GD besteht, sowie wenigstens einen Teilstrom I des Mengenstroms *Ṁ* durch den wenigstens einen Sekundärraum des indirekten Umlaufwärmeaustauschers UW hindurchführt und dabei durch indirekten Wärmeaustausch mit einem gleichzeitig durch den wenigstens einen Primärraum des Umlaufwärmeaustauschers UW hindurchgeführten fluiden Wärmeträger auf eine oberhalb der Temperatur T^{SU} liegende Rückspalttemperatur T^{RS} erwärmt, und von dem aus dem wenigsten einen Sekundärraum des Umlaufwärmeaustauschers UW mit der Temperatur T^{RS} herausgeführten Stoffstroms *Ṁ** an einer Zuführstelle II, die sich unterhalb der untersten trennwirksamen Einbaute der Trennkolonne K und oberhalb des Stands S der Sumpfflüssigkeit befindet, wenigstens einen Teilstrom II in den Sumpfraum der Trennkolonne K so zurückführt, dass der wenigstens eine Teilstrom II im Sumpfraum der Trennkolonne K nicht auf die Sumpfflüssigkeit gerichtet ist, und wenigstens von einem der beiden Ströme *Ṁ, Ṁ** einen Teilstrom als Reststrom ausschleust, mit der Maßgabe, dass die Rückspalttemperatur T^{RS} so bemessen ist, dass einerseits beim Durchströmen des wenigstens einen Sekundärraums des Umlaufwärmeaustauschers UW wenigstens eine Teilmenge der im wenigstens einen Teilstrom I enthaltenen Michael-Addukte unter Ausbildung der zugehörigen Rückspaltprodukte rückgespalten wird, sowie andererseits der in die Trennkolonne K zurückgeführte wenigstens eine Teilstrom II beim an der Zuführstelle II im Sumpfraum vorherrschenden Gasdruck GD siedet und die sich beim Sieden ausbildende, wenigstens eine Teilmenge der Rückspaltprodukte umfassende, Gasphase dem zum Kopfraum der Trennkolonne K hin in der Trennkolonne K abnehmende Gasdruck folgend als Rückspaltprodukt enthaltender Gasstrom G in den Kopfraum der Trennkolonne K strömt, und der Gasstrom G noch im Kopfraum der Trennkolonne K und/oder aus dem Kopfraum der Trennkolonne K herausgeführt durch direkte und/oder indirekte Kühlung teilweise kondensiert, das dabei gebildete Kondensat wenigsten teilweise als Rücklaufflüssigkeit in die Trennkolonne K rückgeführt und der bei der partiellen Kondensation verbleibende Gasstrom ausgelassen wird, dadurch gekennzeichnet, dass die Pumpe P eine Radialkreiselpumpe mit einem halboffenen radialen Laufrad ist.
2. Verfahren gemäß Ausführungsform 1, dadurch gekennzeichnet, dass die in der Flüssigkeit F enthaltenen Michael-Addukte bei der Herstellung eines Esters aus Acrylsäure und einem C₁- bis C₁₀-Alkohol gebildet wurden und die Flüssigkeit F die nachfolgenden Gehalte aufweist:

| | |
|---|---|
| 1 bis 30 Gew.-% | Acrylsäureester, |
| 40 bis 80 Gew.-% | Michael Addukte sowie |
| wenigstens 15 Gew.-% | radikalisches Polymerisat aus Acrylsäure und/oder Acrylsäureester, und |
| 0,1 bis 2 Gew.-% | Polymerisationsinhibitor. |

3. Verfahren gemäß Ausführungsform 1, dadurch gekennzeichnet, dass die in der Flüssigkeit F enthaltenen Michael-Addukte bei der Herstellung von Acrylsäure gebildet wurden und die Flüssigkeit F die nachfolgenden Gehalte aufweist:

| | |
|---|---|
| 10 bis 50 Gew.-% | Michael-Addukte, |
| wenigstens 40 Gew.-% | radikalisches Acrylsäurepolymerisat, |
| bis zu 25 Gew.-% | monomere Acrylsäure, |
| bis zu 2 Gew.-% | Polymerisationsinhibitor, und |
| bis zu 15 Gew.-% | sonstige Verbindungen. |

4. Verfahren gemäß Ausführungsform 1, dadurch gekennzeichnet, dass die in der Flüssigkeit F enthaltenen Michael-Addukte bei der Herstellung von Acrylsäure gebildet wurden und die Flüssigkeit F die nachfolgenden Gehalte aufweist:

| | |
|---|---|
| 10 bis 50 Gew.-% | Michael-Addukte, |
| 40 bis 80 Gew.-% | radikalisches Acrylsäurepolymerisat, |
| 5 bis 20 Gew.-% | monomere Acrylsäure, |
| 0,1 bis 2 Gew.-% | Polymerisationsinhibitor, und |
| 1 bis 15 Gew.-% | sonstige Verbindung. |

5. Verfahren gemäß Ausführungsform 3 oder 4, dadurch gekennzeichnet, dass 40 bis 60 Gew.-% der in der Flüssigkeit F enthaltenen Michael-Addukte Acrylsäure-Michael-Dimere und 15 bis 30 Gew.-% Acrylsäure-Michael-Trimere sind.
6. Verfahren gemäß einer der Ausführungsformen 1 bis 5, dadurch gekennzeichnet, dass die Trennkolonne K Dual-Flow-Boden enthält.
7. Verfahren gemäß einer der Ausführungsformen 1 bis 6, dadurch gekennzeichnet, dass der Umlaufwärmeaustauscher UW mit der Maßgabe als ein Zwangsumlaufentspannungswärmeaustauscher betrieben wird, dass sich zwischen der Zuführstelle II und dem Austritt des Stoffstroms *Ṁ* aus dem wenigstens einen Sekundärraum des Zwangsumlaufentspannungswärmeaustauschers eine Drosselvorrichtung befindet, und in Strömungsrichtung der Arbeitsdruck vor der Drosselvorrichtung größer ist als der Gasdruck GD im Sumpfraum.
8. Verfahren gemäß einer der Ausführungsformen 1 bis 7, dadurch gekennzeichnet, dass zur Unterstützung des Verfahrens der Rückspaltung oberhalb des Stands S der Sumpfflüssigkeit und unterhalb der untersten trennwirksamen Einbaute der Trennkolonne K ein Strippgas in die Trennkolonne K geführt wird.
9. Verfahren gemäß einer der Ausführungsformen 1 bis 8, dadurch gekennzeichnet, dass der Arbeitsdruck am Kopf der Trennkolonne K > 1 bis 3 bar beträgt.
10. Verfahren gemäß einer der Ausführungsformen 1 bis 9, dadurch gekennzeichnet, dass der Umlaufwärmeaustauscher UW ein Rohrbündelwärmeaustauscher ist.
11. Verfahren gemäß Ausführungsform 8, dadurch gekennzeichnet, dass der wenigstens eine Teilstrom II und das Strippgas mittels eines aus einem Innenrohr und aus einem dieses einhüllenden Außenrohr bestehenden koaxialen Doppelrohres in den Sumpfraum geführt werden, wobei das Strippgas im Innenrohr und der wenigstens eine Teilstrom II im Außenrohr geführt wird und das Innenrohr gegenüber dem Außenrohr thermisch isoliert ist.
12. Verfahren gemäß Ausführungsform 11, dadurch gekennzeichnet, dass sowohl das Strippgas als auch der wenigstens eine Teilstrom II aus dem koaxialen Doppelrohr auf eine Prallvorrichtung ausströmen, die beide Ströme nach oben in die Trennkolonne K umlenkt.
13. Verfahren gemäß einer der Ausführungsformen 1 bis 12, dadurch gekennzeichnet, dass die dynamische Viskosität der Sumpfflüssigkeit bei der Temperatur T^{SU} 30 bis 90 beträgt.
14. Verfahren gemäß einer der Ausführungsformen 1 bis 13, dadurch gekennzeichnet, dass der Wirkungsgrad Q des Rückspaltverfahrens wenigstens 20 % beträgt.

US Provisional Patent Application No. 61/122154, eingereicht am 12. Dezember 2008, ist in die vorliegende Anmeldung eingefügt durch Literaturhinweis. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

### Beispiel und Vergleichsbeispiel

Die Elemente der verwendeten Rückspaltvorrichtung waren:
A) Als Pumpe P wurde die Radialkreiselpumpe KSB CPKN-C1.V 200-400 der KSB Aktiengesellschaft in DE-67227 Frankenthal (Pfalz), mit doppelt wirkender Gleitringdichtung und einem Ethylenglycol (40 Gew.-%)/Wasser (60 Gew.-%)-Gemisch als Sperrflüssigkeit verwendet.
   Im Vergleichsbeispiel wurde vorgenannte Radialkreiselpumpe wie von KSB bezogen, d. h., mit geschlossenem radialem Laufrad, eingesetzt.
   Im Beispiel wurde dieselbe Pumpe eingesetzt, jedoch wurde zurvor die Deckscheibe des radialen Laufrads von den Laufradschaufeln entfernt (abgefräßt).
B) Als indirekter Umlaufwärmeaustauscher UW wurde der in der Beschreibung dieser Schrift näher ausgeführte Dreizehnstromrohrbündelwärmeübertrager (bzw.-wärmeaustauscher) D* verwendet (Fertigungsmaterial: Edelstahl vom DIN-Typ 1.4571 rohrseitig und 1.0425 mantelseitig).
C) Die Trennkolonne K und die Verbindungselemente waren wie folgt gestaltet:
   Fertigungsmaterial der Trennkolonne K war Edelstahl vom DIN-Typ 1.4571. Sie war ebenso wie alle Zu- und Ableitungen gegen die Umgebung thermisch isoliert. Als trennwirksamen Einbauten enthielt sie 50 Dual-Flow-Böden (Regensiebböden). Der Innendurchmesser der Trennkolonne K betrug über alle Dual-Flow-Böden einheitlich 2,4 m. Die Dual-Flow-Böden waren in der Strippkolonne äquidistant angeordnet, mit einem lichten Abstand von 400 mm. Ihr Öffnungsverhältnis betrug einheitlich 12 %. Die Lochdurchmesser der Dual-Flow-Böden betrug einheitlich 14 mm (Lochanordnung entsprechend strenger Dreiecksteilung; Abstand von Lochmitte zu Lochmitte = 26 mm (Böden 1 bis 4 von unten), 25,5 mm (Böden 5 bis 8 von unten), 25 mm (Böden 9 bis 49 von unten) sowie 25,5 mm (Boden 50 von unten). Die Bodendicke betrug jeweils 4 mm. Der unterste der Dual-Flow-Böden war 7435 mm oberhalb des unteren Kolonnenendes (d.h., gemessen vom tiefsten Punkt der Kolonne betrug A = 7435 mm) angebracht. Oberhalb des letzten Dual-Flow-Bodens war ein Kaminboden als Sammelboden angebracht. Die Oberkante der Kamine dieses Sammelbodens befand sich 29525 mm oberhalb des unteren Kolonnenendes. Die Kamine waren bedacht und wiesen einen Innendruchmesser von 316,7 mm und eine Höhe (gerechnet bis Überlaufhöhe ohne Hut) von 1030 mm auf. Ihre Gesamtzahl betrug 12 und war über den Kaminboden gleichförmig verteilt. Der Sammelboden war einfachwandig mit 2° Gefälle nach außen und mit seitlichem Abzug und Abzugstutzen (DN 200) gestaltet. Der freie Gasquerschnitt betrug ca. 30 %. 4940 mm oberhalb der Kaminoberkante (ohne Hut gerechnet) waren durch die Kolonnenwand radial in den Kopfraum der Trennkolonne K (der Kaminboden bildete die Verbindung zwischen Kopfraum und Trennraum der Trennkolonne K) sechs Rohre eingeführt, deren Innendurchmesser 82 mm und deren Wanddicke 2,6 mm betrug. Über den Umfang der Kolonne waren die Einführstellen der Rohre äquidistant verteilt (Einschlusswinkel zweier benachbarter Rohre = 60°).

   In einer Entfernung von 500 mm von der Innenwand der Kolonne waren fünf der sechs Rohre nach unten gekrümmt und endeten in einer kreisförmigen Düsenöffnung mit einem Innendurchmesser von 2,5 Zoll.
   Das sechste Rohr hatte eine von der Kolonneninnenwand radial ins Kolonneninnere reichende Länge von 800 mm. Im Abstand von 500 mm von der Kolonnenwand wies es eine nach unten weisende kreisförmige Düsenöffnung mit einem Innendurchmesser von ebenfalls 2,5 Zoll auf. Am Längenende wies dieses Rohr eine zusätzlich kreisförmige Düsenöffnung mit einem Innendurchmesser von 1¼ Zoll auf. Der Zentralstrahl des zugehörigen Sprühkegels wies eine nach oben gerichtete Vektorkomponente auf und schloss mit der Vertikalen zum Kolonnenquerschnitt einen Winkel von 15° ein.
   Über eine außerhalb der Kolonne angebrachte Ringleitung, an die die sechs Rohre angeschlossen waren, wurden die sechs Rohre mit der Flüssigkeit (zuvor gebildetes Kondensat) zur Direktkühlung des durch den Kaminboden in den Kopfraum der Trennkolonne K strömenden Gasstroms G versorgt und diese Flüssigkeit ins Kolonneninnere versprüht. Die Länge der Trennkolonne K (gemessen vom tiefstgelegenen Punkt des Sumpfraums bis zum höchstgelegenen Punkt des Kopfraums) betrug 35260 mm.
   Am höchstgelegenen Punkt des Kopfraums befand sich der Auslass DN 500 (Innendurchmesser = 498 mm) für den bei der Direktkühlung des Gasstroms G verbleibenden Gasstrom.
   Am tiefstgelegenen Punkt des Sumpfraums befand sich der Auslass DN 400 (Innendurchmesser = 398,4 mm) für den Mengenstrom *Ṁ*. Er war mit einem Trombenbrecher ausgestattet.
   Die Verbindung von Radialkreiselpumpe und Dreizehnstromrohrbündelwärmeübertrager war in der nachstehenden Abfolge zum Wärmeübertrager hin aufeinander folgend
   1 Bogen DN 200 (90°, Radius: 305 mm);
   1 Konus DN 200 auf DN 300 (Länge: 203 mm);
   1 gerades Rohr DN 300 (Länge: 600 mm);
   1 Bogen DN 300 (90°, Radius 457 mm);
   1 gerades Rohr DN 300 (Länge: 900 mm);
   1 Bogen DN 300 (90°; Radius 457 mm);
   1 gerades Rohr DN 300 (Länge: 2800 mm);
   1 Reduzierung DN 300 auf DN 250 (Länge: 203 mm);
   2 Bögen DN 250 (90°, Radius: 381 mm);
   1 gerades Rohr DN 250 (Länge: 900 mm);
   1 Bogen DN 250 (90°, Radius 381 mm);
   1 gerades Rohr DN 250 (Länge: 6000 mm);
   1 Bogen DN 250 (90°, Radius: 381 mm);
   1 gerades Rohr DN 250 (Länge: 800 mm);
   1 Reduzierung DN 250 auf DN 300 (Länge: 203 mm); und
   1 Bogen DN 300 (90°, Radius 457 mm).

   Die Verbindung vom Auslass der Trennkolonne K für den Mengenstrom M zum Dreizehnstromrohrbündelwärmeübertrager war in der nachstehenden Abfolge zum Wärmeübertrager hin aufeinanderfolgend
   1 Rohrleitungskompensator DN 400 (Länge ca. 510 mm);
   1 gerades Rohr DN 400 (Länge: ca. 2400 mm);
   1 Bogen DN 400 (ca. 15°, Radius: 610 mm);
   1 Bogen DN 400 (90°, Radius: 600 mm);
   1 gerades Rohr DN 400 (Länge: ca. 5000 mm); und
   1 Reduzierung DN 400 auf DN 250 (Länge: 350 mm).

   Zwischen dem Austritt des Stoffstroms *Ṁ** aus dem Dreizehnstromrohrbündelwärmeübertrager und dem Sumpfraum der Trennkolonne K war als Drosselvorrichtung eine Lochblende angebracht.
   Der Wiedereintritt des überhitzten "Teilstroms" II in den Sumpfraum der Trennkolonne K war als in die Querschnittsmitte des Sumpfraums gezogenes koaxiales Doppelrohr (gegeneinander thermisch isolierte Rohre) ausgeführt, wo es nach unten gekrümmt auf den Spiegel der Sumpfflüssigkeit zeigte. In Strömungsrichtung hinter dem Rohrende war, an der äußeren Rohreinhüllenden befestigt, eine Prallvorrichtung angebracht, die den auf sie aufprallenden Austrittsstrom nach oben umlenkte. Im äußeren Ring des koaxialen Doppelrohrs wurde die überhitzte Sumpfflüssigkeit geführt, im Kern des koaxialen Doppelrohres wurde gleichzeitig das Strippgas ("Unterstützungsgas") zudosiert.
   Für die Rücklaufflüssigkeit befand sich 770 mm oberhalb des obersten Dual-Flow-Bodens und unterhalb des Kaminbodens ein zu einem geschlossenen Kreis ausgeführtes Verteilerrohr (in die Kolonne zentriert sowie waagrecht, d. h., parallel zum Kolonnenquerschnitt, eingebracht) dem die Rücklaufflüssigkeit zugeführt wurde. Der Kreisinnendurchmesser betrug 1870 mm. Der Rohraußendurchmesser betrug 33,7 mm und der Rohrinnendurchmesser war 25 mm. Das kreisförmig ausgeführte Verteilerrohr wies 21 Lochöffnungen auf, deren Innendurchmesser 5 mm betrug. Jede zweite dieser Öffnungen war auf die Unterseite des Kaminbodens gerichtet, um diesen mit Rücklaufflüssigkeit feucht zu halten. Der Zentralstrahl aus der anderen Hälfte der Öffnungen wies in einem Winkel von 45° (zur Senkrechten auf den Kolonnenquerschnitt) hälftig nach unten in die Kolonnemitte sowie hälftig nach unten zur Kolonnenwand.
   Zusätzlich waren im Umfang des kreisförmig ausgeführten Verteilerrohres drei über den Umfang gleichmäßig verteilte (Einschlusswinkel 120°) und radial nach außen weisende Präzisionsstrahlrohre (Länge = 200 mm, Außendurchmesser = 6 mm, Innendurchmesser = 4 mm) angebracht. Der Austritt aus dem Präzisionsstrahlrohren war auf Kugelhähne gerichtet, über die der Kolonne Waschflüssigkeit zugeführt werden konnte. Über die Lochöffnungen und die Strahlrohre wurde die Rücklaufflüssigkeit dem trennwirksamen Teil zugeführt.
   Zwischen dem achten und dem neunten Dual-Flow-Boden (von unten nach oben gezählt) befand sich ein Zulaufstutzen, über den die dem Spaltverfahren zu unterwerfende Flüssigkeit F in die Trennkolonne K geführt wurde. Die Zufuhr war dabei als ein in den Zulaufstutzen geführtes Einsteckrohr ausgestaltet, das auf seiner Unterseite Bohrungen aufwies, über die die Flüssigkeit F gleichmäßig auf den darunter liegenden Boden aufgegeben wurde.

In der Rückspaltvorrichtung wurde die Rückspaltung für die beispielhafte Ausführung im stationären Zustand wie folgt betrieben:
Das Produktgasgemisch einer zweistufigen heterogen katalysierten partiellen gasphasenoxidation von Propylen (chemical grade) zu Acrylsäure, die wie in der beispielhaften Ausführung der WO 2008/090190 beschrieben durchgeführt wurde, wurde wie in der beispielhaften Ausführung der WO 2008/090190 einer fraktionierenden Kondensation unterworfen, um die im Produktgasgemisch der Partialoxidation enthaltene Acrylsäure aus selbigem abzutrennen.

Aus dem Sumpfbereich der Kondensationskolonne wurde wie in der WO 2008/090190 beschrieben Schwersiederflüssigkeit entnommen, die folgende Gehalte aufwies:

| | |
|---|---|
| monomere Acrylsäure | 67,88 Gew.-%, |
| Michael-Diacrylsäure (2AS) | 19,72 Gew.-%, |
| Michael-Triacrylsäure (3AS) | 1,96 Gew.-%, |
| Michael-4AS | 0,34 Gew.-%, |
| Michael-5AS | 0,77 Gew.-%, |
| Michael-6AS | 0,01 Gew.-%, |
| Michael-7AS | 0,01 Gew.-%, |
| Michael-8AS | 0,03 Gew.-%, |
| Michael-9AS | 0,01 Gew.-%, |
| Michael-10AS | 0,01 Gew.-%, |
| radikalisches Acrylsäurepolymerisat | 8,55 Gew.-%, |
| Fumarsäure | 0,27 Gew.-%, |
| Maleinsäure | 0,27 Gew.-%, |
| Phthalsäure | 0,11 Gew.-%, und |
| MEHQ + PTZ | 0,06 Gew.-%. |

3028 kg/h dieser Schwersiederflüssigkeit wurden mit einer Temperatur von 102 °C der Trennkolonne K der Rückspaltvorrichtung zugeführt (auf den 8ten Boden von unten).

Aus dem Sumpfraum der Trennkolonne K wurde durch die Pumpe P ein Mengenstrom *M* mit einer Temperatur T^{SU} von 169 °C angesaugt (seine dynamische Viskosität betrug 60 mPas). Der Stand S der Sumpfflüssigkeit war auf 82 cm eingependelt.

Der Mengenstrom M wies folgende Gehalte auf:

| | |
|---|---|
| monomere Acrylsäure | 9,3 Gew.-%, |
| Michael-Diacrylsäure (2AS) | 10,62 Gew.-%, |
| Michael-Triacrylsäure (3AS) | 5,19 Gew.-%, |
| Michael-4AS | 2,69 Gew.-%, |
| Michael-5AS | 3,38 Gew.-%, |
| Michael-6AS | 0,1 Gew.-%, |
| Michael-7AS | 0,1 Gew.-%, |
| Michael-8AS | 0,2 Gew.-%, |
| Michael-9AS | 0,1 Gew.-%, |
| Michael-10AS | 0,1 Gew.-%, |
| Radikalisches Polymerisat | 63,14 Gew.-%, |
| Fumarsäure | 2 Gew.-% |
| Maleinsäure | 1,96 Gew.-%, |
| Phthalsäure | 0,78 Gew.-%, und |

MEHQ, PTZ und Folgeprodukte als Restmenge bis zu 100 Gew.-%.

Ein Teilstrom von 410 kg/h des Mengenstroms *Ṁ* wurde als Reststrom ausgelassen. Der verbleibende Teilstrom des Mengenstroms *Ṁ* wurde mit der Pumpe P als Teilstrom I (225 m³/h) durch die Rohre des indirekten Umlaufwärmeaustauschers UW gepumpte. Gleichzeitig wurden auf der Sekundärraumseite des Umlaufwärmeaustauschers 1700 kg/h Wasserdampf (19 bar absolut, 210 °C) als Wärmeträger zugeführt.

Der Eintritt des Wasserdampfs und der Eintritt des Teilstroms I in den Dreizehnstromrohrbündelwärmeübertrager befanden sich anwendungstechnisch günstig auf derselben Seite des Wärmeübertragers. Der Arbeitsdruck in Strömungsrichtung vor der Drosselvorrichtung (vor der Blende) betrug 3 bar, während der Gasdruck GD im Sumpfraum 1,7 bar betrug. Die Temperatur des aus der Primärraumseite des Umlaufwärmeaustauschers in den Gasraum des Sumpfraumes rückgeführten Stoffstroms betrug in Strömungsrichtung hinter der Drosselvorrichtung kurz vor seinem Austritt aus dem koaxialen Doppelrohr 171 °C.
Am Kopf der Trennkolonne K wurde ein Gasstrom von 21055 kg/h mit einer Temperatur von 67 °C und einem Druck von 1, 6 bar absolut ausgelassen. Die zugeführte Strippgasmenge (1,7 bar, 148 °C) betrug 18437 kg/h. Bei diesem handelte es sich um vom Kolonnenkopf der Kondensationskolonne entnommenes Restgas das (gemeinsam mit Kreisgas) mit Hilfe eines mehrstufigen Radialverdichters auf seinen Arbeitsdruck verdichtet worden war und zu ca. 88 Gew.-% aus molekularem Stickstoff, zu ca. 3 Gew.-% aus Wasser, zu ca. 5 Gew.-% aus molekularem Stickstoff und zu ca. 2 Gew.% aus Kohlenoxiden bestand. Die Rücklaufflüssigkeit wies eine Temperatur von 67 °C auf und wurde in einem Mengenstrom von 6500 kg/h dem obersten Dual-Flow-Boden zugeführt. Sie bestand im Wesentlichen aus aus dem Gasstrom G auskondensierter Acrylsäure (ca. 90 Gew.-%) und aus Wasser (ca. 6 Gew.-%). Der nicht als Rücklaufflüssigkeit verwendete Restmengenstrom des vom Sammelboden abgeführten Kondensatstroms wurde, aus Gründen der Polymerisationsinhibierung im Gemisch mit einem kleinen Strom von PTZ und MEQ enthaltender, aus dem Sumpf der Kondensationskolonne entnommener Sumpfflüssigkeit durch einen mit Wasser (Eintrittstemperatur = 20°C) gekühlten (im Gegenstrom) Spiralwärmeaustauscher geführt, und dabei auf 32°C abgekühlt. Zum Zweck der Direktkühlung des durch den Kaminboden in den Kopfraum der Kolonne aufsteigenden, mit Acrylsäure beladenen, Gasstroms G wurde die Gesamtmenge des so indirekt abgekühlten flüssigen Gemischstroms über den beschriebenen doppelten Ringverteiler in den Kopfraum der Trennkolonne K versprüht.

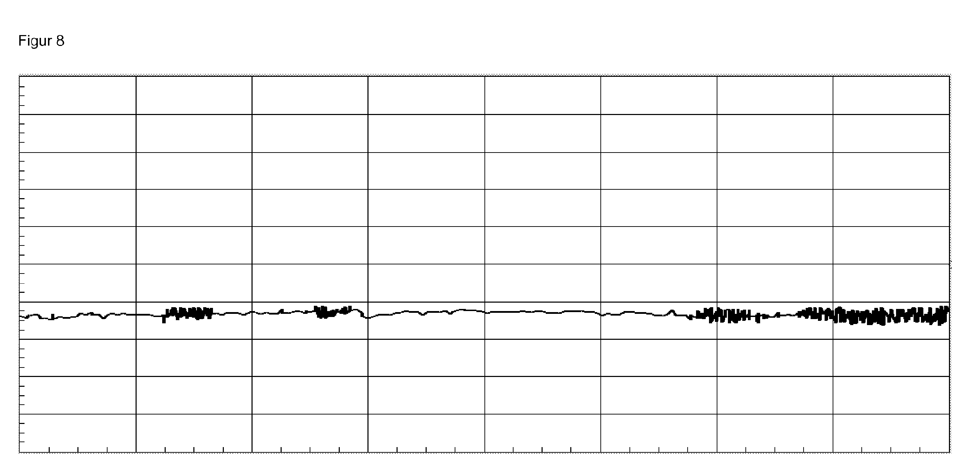

Figur 8 zeigt über einen Ausschnitt von 7 Betriebstagen (Abszisse; Schnittpunkt mit der Ordinate = Beginn der 7-Tage-Periode; rechtes Ende der Abzisse = Ende der 7-Tage-Periode) den Verlauf des durch die verwendete Radialkreiselpumpe mit halboffenem Laufrad im Kreis geführten Mengenstroms (Ordinate; im wesentlichen konstant 225 m³/h). Der Wirkungsgrad Q des Rückspaltverfahrens betrug [(*ṁ*ₑᵢₙ - *ṁ*ₐᵤₛ)/*ṁ*ₑᵢₙ)] × 100% = [(692,8 kg/h - 92,2 kg/h) / 692,8 kg/h] x 100 % = 86,70%.

Für das Vergleichsbeispiel wurde die Rückspaltung wie folgt betrieben:
Gleiches Vorgehen in derselben Rückspaltvorrichtung, jedoch mit dem Unterschied, dass jetzt dieselbe Radialkreiselpumpe aber mit geschlossenem Laufrad verwendet wurde (gleiche Einstellung des Antriebmotors).

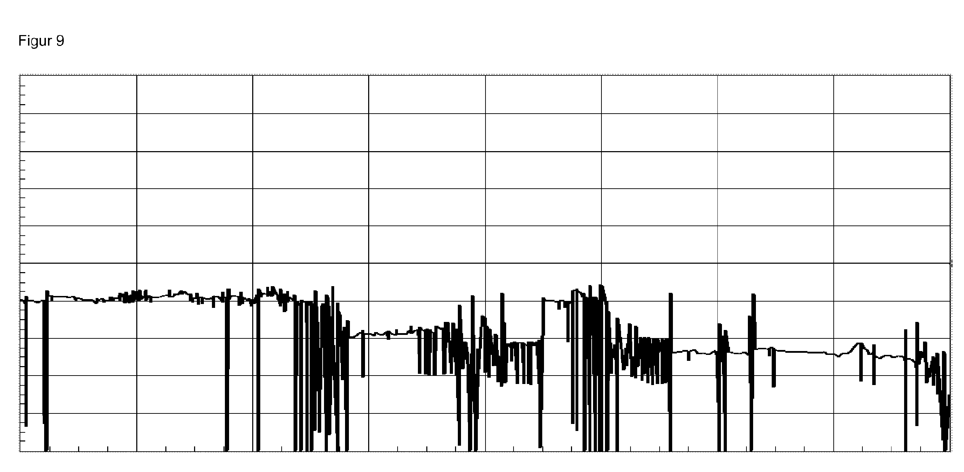

Figur 9 zeigt für diesen Fall wieder über einen Ausschnitt von 7 Betriebstagen den Verlauf des durch die Radialkreiselpumpe mit geschlossenem Laufrad im Kreis geführten Mengenstroms (der Startpunkt auf der Ordinate liegt bei ca. 240 Nm³/h).

Die Ordinate der Figuren 8 und 9 sind identisch skaliert.

Die unregelmäßig auftretenden Leistungseinbrüche sind deutlich sichtbar.

## Patentansprüche

1. Verfahren zur Rückspaltung von in einer Flüssigkeit F mit einem Gewichtsanteil, bezogen auf das Gewicht der Flüssigkeit F, von ≥ 10 Gew.-% enthaltenen Michael-Addukten, die bei der Herstellung von Acrylsäure oder deren Ester gebildet wurden, in einer Rückspaltvorrichtung, die wenigstens eine Pumpe P, eine Trennkolonne K, die von unten nach oben aus einem Sumpfraum, einem sich an den Sumpfraum anschließenden, trennwirksame Einbauten enthaltenden, Trennraum und einem sich an diesen anschließenden Kopfraum besteht und in der der Druck in der Gasphase von unten nach oben abnimmt, sowie einen indirekten Umlaufwärmeaustauscher UW, der wenigstens einen Sekundärraum und wenigstens einen, von dem wenigstens einen Sekundärraum durch eine materielle Trennwand D getrennten, Primärraum aufweist, umfasst, bei dem man kontinuierlich die Flüssigkeit F mit der Zuführtemperatur T^{Z} an einer Zuführstelle I, die sich oberhalb der untersten trennwirksamen Einbaute in der Trennkolonne K befindet, in die Trennkolonne K hineinführt, und an der tiefstgelegenen Stelle des Sumpfraums der Trennkolonne K mit der Pumpe P kontinuierlich einen Mengenstrom *Ṁ* der durch die trennwirksamen Einbauten in den Sumpfraum ablaufenden, Michael-Addukte enthaltenden, Flüssigkeit mit einer Temperatur T^{SU} entnimmt, so dass sich im Sumpfraum als Sumpfflüssigkeit ein Stand S der in diesen ablaufenden Flüssigkeit einstellt, der weniger als der halbe Abstand A, gemessen vom tiefstgelegenen Punkt der Trennkolonne K bis zur Unterseite der untersten trennwirksamen Einbaute in der Tennkolonne K, beträgt, während im über diesem Flüssigkeitsstand liegenden restlichen Raum des Sumpfraums ein Gasdruck GD besteht, sowie wenigstens einen Teilstrom I des Mengenstroms *Ṁ* durch den wenigstens einen Sekundärraum des indirekten Umlaufwärmeaustauschers UW hindurchführt und dabei durch indirekten Wärmeaustausch mit einem gleichzeitig durch den wenigstens einen Primärraum des Umlaufwärmeaustauschers UW hindurchgeführten fluiden Wärmeträger auf eine oberhalb der Temperatur T^{SU} liegende Rückspalttemperatur T^{RS} erwärmt, und von dem aus dem wenigsten einen Sekundärraum des Umlaufwärmeaustauschers UW mit der Temperatur T^{RS} herausgeführten Stoffstroms *Ṁ** an einer Zuführstelle II, die sich unterhalb der untersten trennwirksamen Einbaute der Trennkolonne K und oberhalb des Stands S der Sumpfflüssigkeit befindet, wenigstens einen Teilstrom II in den Sumpfraum der Trennkolonne K so zurückführt, dass der wenigstens eine Teilstrom II im Sumpfraum der Trennkolonne K nicht auf die Sumpfflüssigkeit gerichtet ist, und wenigstens von einem der beiden Ströme *Ṁ*, *Ṁ** einen Teilstrom als Reststrom ausschleust, mit der Maßgabe, dass die Rückspalttemperatur T^{RS} so bemessen ist, dass einerseits beim Durchströmen des wenigstens einen Sekundärraums des Umlaufwärmeaustauschers UW wenigstens eine Teilmenge der im wenigstens einen Teilstrom I enthaltenen Michael-Addukte unter Ausbildung der zugehörigen Rückspaltprodukte rückgespalten wird, sowie andererseits der in die Trennkolonne K zurückgeführte wenigstens eine Teilstrom II beim an der Zuführstelle II im Sumpfraum vorherrschenden Gasdruck GD siedet und die sich beim Sieden ausbildende, wenigstens eine Teilmenge der Rückspaltprodukte umfassende, Gasphase dem zum Kopfraum der Trennkolonne K hin in der Trennkolonne K abnehmende Gasdruck folgend als Rückspaltprodukt enthaltender Gasstrom G in den Kopfraum der Trennkolonne K strömt, und der Gasstrom G noch im Kopfraum der Trennkolonne K und/oder aus dem Kopfraum der Trennkolonne K herausgeführt durch direkte und/oder indirekte Kühlung teilweise kondensiert, das dabei gebildete Kondensat wenigsten teilweise als Rücklaufflüssigkeit in die Trennkolonne K rückgeführt und der bei der partiellen Kondensation verbleibende Gasstrom ausgelassen wird, **dadurch gekennzeichnet, dass** die Pumpe P eine Radialkreiselpumpe mit einem halboffenen radialen Laufrad ist.

2. Verfahren nach Anspruch 1, **dadurch gekennzeichnet, dass** die in der Flüssigkeit F enthaltenen Michael-Addukte bei der Herstellung eines Esters aus Acrylsäure und einem C₁- bis C₁₀-Alkohol gebildet wurden und die Flüssigkeit F die nachfolgenden Gehalte aufweist:
| | |
|---|---|
| 1 bis 30 Gew.-% | Acrylsäureester, |
| 40 bis 80 Gew.-% | Michael Addukte sowie |
| wenigstens 15 Gew.-% | radikalisches Polymerisat aus Acrylsäure und/oder Acrylsäureester, und |
| 0,1 bis 2 Gew.-% | Polymerisationsinhibitor. |

3. Verfahren nach Anspruch 1, **dadurch gekennzeichnet, dass** die in der Flüssigkeit F enthaltenen Michael-Addukte bei der Herstellung von Acrylsäure gebildet wurden und die Flüssigkeit F die nachfolgenden Gehalte aufweist:
| | |
|---|---|
| 10 bis 50 Gew.-% | Michael-Addukte, |
| wenigstens 40 Gew.-% | radikalisches Acrylsäurepolymerisat, |
| bis zu 25 Gew.-% | monomere Acrylsäure, |
| bis zu 2 Gew.-% | Polymerisationsinhibitor, und |
| bis zu 15 Gew.-% | sonstige Verbindungen. |

4. Verfahren nach Anspruch 1, **dadurch gekennzeichnet, dass** die in der Flüssigkeit F enthaltenen Michael-Addukte bei der Herstellung von Acrylsäure gebildet wurden und die Flüssigkeit F die nachfolgenden Gehalte aufweist:
| | |
|---|---|
| 10 bis 50 Gew.-% | Michael-Addukte, |
| 40 bis 80 Gew.-% | radikalisches Acrylsäurepolymerisat, |
| 5 bis 20 Gew.-% | monomere Acrylsäure, |
| 0,1 bis 2 Gew.-% | Polymerisationsinhibitor, und |
| 1 bis 15 Gew.-% | sonstige Verbindung. |

5. Verfahren nach Anspruch 3 oder 4, **dadurch gekennzeichnet, dass** 40 bis 60 Gew.-% der in der Flüssigkeit F enthaltenen Michael-Addukte Acrylsäure-Michael-Dimere und 15 bis 30 Gew.-% Acrylsäure-Michael-Trimere sind.

6. Verfahren nach einem der Ansprüche 1 bis 5, **dadurch gekennzeichnet, dass** die Trennkolonne K Dual-Flow-Boden enthält.

7. Verfahren nach einem der Ansprüche 1 bis 6, **dadurch gekennzeichnet, dass** der Umlaufwärmeaustauscher UW mit der Maßgabe als ein Zwangsumlaufentspannungswärmeaustauscher betrieben wird, dass sich zwischen der Zuführstelle II und dem Austritt des Stoffstroms M aus dem wenigstens einen Sekundärraum des Zwangsumlaufentspannungswärmeaustauschers eine Drosselvorrichtung befindet, und in Strömungsrichtung der Arbeitsdruck vor der Drosselvorrichtung größer ist als der Gasdruck GD im Sumpfraum.

8. Verfahren nach einem der Ansprüche 1 bis 7, **dadurch gekennzeichnet, dass** zur Unterstützung des Verfahrens der Rückspaltung oberhalb des Stands S der Sumpfflüssigkeit und unterhalb der untersten trennwirksamen Einbaute der Trennkolonne K ein Strippgas in die Trennkolonne K geführt wird.

9. Verfahren nach einem der Ansprüche 1 bis 8, **dadurch gekennzeichnet, dass** der Arbeitsdruck am Kopf der Trennkolonne K > 1 bis 3 bar beträgt.

10. Verfahren nach einem der Ansprüche 1 bis 9, **dadurch gekennzeichnet, dass** der Umlaufwärmeaustauscher UW ein Rohrbündelwärmeaustauscher ist.

11. Verfahren nach Anspruch 8, **dadurch gekennzeichnet, dass** der wenigstens eine Teilstrom II und das Strippgas mittels eines aus einem Innenrohr und aus einem dieses einhüllenden Außenrohr bestehenden koaxialen Doppelrohres in den Sumpfraum geführt werden, wobei das Strippgas im Innenrohr und der wenigstens eine Teilstrom II im Außenrohr geführt wird und das Innenrohr gegenüber dem Außenrohr thermisch isoliert ist.

12. Verfahren nach Anspruch 11, **dadurch gekennzeichnet, dass** sowohl das Strippgas als auch der wenigstens eine Teilstrom II aus dem koaxialen Doppelrohr auf eine Prallvorrichtung ausströmen, die beide Ströme nach oben in die Trennkolonne K umlenkt.

13. Verfahren nach einem der Ansprüche 1 bis 12, **dadurch gekennzeichnet, dass** die dynamische Viskosität der Sumpfflüssigkeit bei der Temperatur T^{SU} 30 bis 90 beträgt.

14. Verfahren nach einem der Ansprüche 1 bis 13, **dadurch gekennzeichnet, dass** der Wirkungsgrad Q des Rückspaltverfahrens wenigstens 20 % beträgt.

## Claims

1. A process for redissociating Michael adducts which are present in a liquid F with a proportion by weight, based on the weight of the liquid F, of ≥ 10% by weight and have been formed in the preparation of acrylic acid or esters thereof in a redissociating apparatus which comprises at least one pump P, a separating column K which consists, from the bottom upward, of a bottom space, a separating space which adjoins the bottom space and comprises separating internals, and a top space which adjoins the separating space, and in which the pressure in the gas phase decreases from the bottom upward, and an indirect circulation heat exchanger UW which has at least one secondary space and at least one primary space separated from the at least one secondary space by a material dividing wall D, in which the liquid F is conducted continuously into the separating column K with the feed temperature T^{Z} at a feed point I which is above the lowermost separating internal in the separating column K, and, at the lowest point in the bottom space of the separating column K, the pump P is used to continuously withdraw a mass flow *Ṁ* of the liquid which effluxes into the bottom space through the separating internals and comprises Michael adducts with a temperature T^{SU} so as to establish, in the bottom space, as the bottoms liquid, a level S of the liquid effluxing into it which is less than half the distance A, measured from the lowest point in the separating column K to the underside of the lowermost separating internal in the separating column K, while a gas pressure GD exists in the remaining space of the bottom space above this liquid level, and at least one substream I of the mass flow *Ṁ* is conducted through the at least one secondary space of the indirect circulation heat exchanger UW while being heated, by indirect heat exchange with a fluid heat carrier conducted simultaneously through the at least one primary space of the circulation heat exchanger UW, to a redissociation temperature T^{RS} above the temperature T^{SU}, and at least one substream II from the mass flow *Ṁ** conducted out of the at least one secondary space of the circulation heat exchanger UW with the temperature T^{RS} is recycled into the bottom space of the separating column K at a feed point II which is below the lowermost separating internal of the separating column K and above the level S of the bottoms liquid, in such a way that the at least one substream II in the bottom space of the separating column K is not directed toward the bottoms liquid, and a substream at least of one of the two streams *Ṁ, Ṁ* * is discharged as a residual stream, with the proviso that the redissociation temperature T^{RS} is such that, firstly, as it flows through the at least one secondary space of the circulation heat exchanger UW, at least a portion of the Michael adducts present in the at least one substream I is redissociated to form the corresponding redissociation products, and, secondly, the at least one substream II recycled into the separating column K boils at the gas pressure GD existing in the bottom space at the feed point II, and the gas phase which forms in the course of boiling and comprises at least a portion of the redissociation products, following the gas pressure which decreases in the separating column K toward the top space of the separating column K, flows as a gas stream G comprising redissociation products into the top space of the separating column K and the gas stream G is partly condensed by direct and/or indirect cooling still in the top space of the separating column K and/or conducted out of the top space of the separating column K, the condensate formed is recycled at least partly as reflux liquid into the separating column K and the gas stream which remains in the partial condensation is discharged, wherein the pump P is a radial centrifugal pump with a semiopen radial impeller.

2. The process according to claim 1, wherein the Michael adducts present in the liquid F have been formed in the preparation of an ester from acrylic acid and a C₁- to C₁₀-alcohol, and the liquid F has the following contents:
1 to 30% by weight of acrylic ester,
40 to 80% by weight of Michael adducts and
at least 15% by weight of free-radical polymer of acrylic acid and/or acrylic ester, and
0.1 to 2% by weight of polymerization inhibitor.

3. The process according to claim 1, wherein the Michael adducts present in the liquid F have been formed in the preparation of acrylic acid and the liquid F has the following contents:
10 to 50% by weight of Michael adducts,
at least 40% by weight of free-radical acrylic acid polymer,
up to 25% by weight of monomeric acrylic acid,
up to 2% by weight of polymerization inhibitor, and
up to 15% by weight of other compounds.

4. The process according to claim 1, wherein the Michael adducts present in the liquid F have been formed in the preparation of acrylic acid and the liquid F has the following contents:
10 to 50% by weight of Michael adducts,
40 to 80% by weight of free-radical acrylic acid polymer,
5 to 20% by weight of monomeric acrylic acid,
0.1 to 2% by weight of polymerization inhibitor, and
1 to 15% by weight of other compounds.

5. The process according to claim 3 or 4, wherein 40 to 60% by weight of the Michael adducts present in the liquid F are Michael acrylic acid dimers and 15 to 30% by weight are Michael acrylic acid trimers.

6. The process according to any one of claims 1 to 5, wherein the separating column K comprises dual-flow trays.

7. The process according to any one of claims 1 to 6, wherein the circulation heat exchanger UW is operated as forced circulation flash heat exchanger with the proviso that a throttle apparatus is present between the feed point II and the exit of the mass flow *Ṁ* from the at least one secondary space of the forced circulation flash heat exchanger, and the working pressure upstream of the throttle apparatus is greater than the gas pressure GD in the bottom space in flow direction.

8. The process according to any one of claims 1 to 7, wherein the redissociation process is promoted by conducting a stripping gas into the separating column K above the level S of the bottoms liquid and below the lowermost separating internal of the separating column K.

9. The process according to any one of claims 1 to 8, wherein the working pressure at the top of the separating column K is > 1 to 3 bar.

10. The process according to any one of claims 1 to 9, wherein the circulation heat exchanger UW is a tube bundle heat exchanger.

11. The process according to claim 8, wherein the at least one substream II and the stripping gas are conducted into the bottom space by means of a coaxial double tube consisting of an inner tube and of an outer tube enclosing the latter, the stripping gas being conducted within the inner tube and the at least one substream II in the outer tube, and the inner tube having been thermally insulated from the outer tube.

12. The process according to claim 11, wherein both the stripping gas and the at least one substream II flow out of the coaxial double tube onto a baffle device which deflects both streams upward into the separating column K.

13. The process according to any one of claims 1 to 12, wherein the dynamic viscosity of the bottoms liquid at the temperature T^{SU} is 30 to 90.

14. The process according to any one of claims 1 to 13, wherein the efficiency Q of the redissociation process is at least 20%.

## Revendications

1. Procédé pour la dissociation en retour de produits d'addition de Michaël contenus dans un liquide F à une proportion pondérale, par rapport au poids du liquide F, ≥ 10 % en poids, qui ont été formés lors de la préparation d'acide acrylique ou de ses esters, dans un dispositif de dissociation en retour, qui comprend au moins une pompe P, une colonne de séparation K, qui est constituée, de bas en haut, d'un espace de fond, d'un espace de séparation consécutif à l'espace de fond, contenant des encastrements actifs en séparation, et d'un espace de tête consécutif et dans laquelle la pression dans la phase gazeuse diminue de bas en haut, ainsi qu'un échangeur thermique à circulation indirect UW, qui présente au moins un espace secondaire et au moins un espace primaire, séparé dudit au moins un espace secondaire par une paroi de séparation D matérielle, dans lequel on introduit en continu, dans la colonne de séparation K, le liquide F à la température d'alimentation T^{Z} au niveau d'un site d'alimentation I, qui se trouve au-dessus de l'encastrement actif en séparation inférieur dans la colonne de séparation K, et on prélève en continu au niveau du site le plus bas de l'espace de fond de la colonne de séparation K, à l'aide de la pompe P, un flux massique M du liquide qui s'écoule en descendant à travers les encastrements actifs en séparation dans l'espace de fond, contenant des produits d'addition de Michaël, présentant une température T^{su}, de telle sorte qu'il se règle, dans l'espace de fond, comme liquide de fond, un niveau S du liquide qui s'écoule en descendant dans celui-ci, qui est inférieur à la demi distance A, mesurée à partir du point le plus bas de la colonne de séparation K jusqu'à la face inférieure de l'encastrement actif en séparation inférieur dans la colonne de séparation K, alors qu'il existe dans l'espace qui reste au-dessus de ce niveau de liquide de l'espace de fond une pression gazeuse GD, et on fait passer au moins un flux partiel I du flux massique M à travers ledit au moins un espace secondaire de l'échangeur thermique à circulation indirect UW et on le chauffe ainsi, par échange thermique indirect avec un caloporteur fluide qu'on fait passer simultanément à travers ledit au moins un espace primaire de l'échangeur thermique à circulation UW, à une température de dissociation en retour T^{RS} supérieure à la température T^{SU} et on recycle, du flux massique Ṁ * évacué dudit au moins un espace secondaire de l'échangeur thermique à circulation UW présentant la température T^{RS}, au niveau d'un site d'alimentation II, qui se trouve sous l'encastrement actif en séparation inférieur de la colonne de séparation K et au-dessus du niveau S, au moins un flux partiel II dans l'espace de fond de la colonne de séparation K de manière telle que ledit au moins un flux partiel II, dans l'espace de fond de la colonne de séparation K, n'est pas dirigé sur le liquide de fond et on soutire d'au moins un des deux flux Ṁ, Ṁ * un flux partiel comme flux résiduel, à condition que la température de dissociation en retour T^{RS} soit dimensionnée de manière telle que d'une part, lors de l'écoulement à travers ledit au moins un espace secondaire de l'échangeur thermique à circulation UW, au moins une quantité partielle des produits d'addition de Michaël contenus dans ledit au moins un flux partiel I est dissociée en retour avec formation des produits de dissociation en retour associés et que d'autre part ledit au moins un flux partiel II recyclé dans la colonne de séparation K bout à la pression gazeuse GD qui règne au niveau du site d'alimentation II dans l'espace de fond, et la phase gazeuse qui se forme lors de l'ébullition, comprenant au moins une quantité partielle des produits dissociés en retour, suivant la pression gazeuse qui diminue dans la colonne de séparation K vers l'espace de tête de la colonne de séparation K, s'écoule comme flux gazeux G contenant le produit de dissociation en retour dans l'espace de tête de la colonne de séparation K et le flux gazeux G est partiellement condensé lorsqu'il est encore dans l'espace de tête de la colonne de séparation K et/ou lorsqu'il est évacué de l'espace de tête de la colonne de séparation K par refroidissement direct et/ou indirect, le condensat formé est recyclé au moins partiellement comme liquide de reflux dans la colonne de séparation K et le flux gazeux qui reste lors de la condensation partielle est évacué, **caractérisé en ce que** la pompe P est une pompe centrifuge radiale présentant une roue radiale semi-ouverte.

2. Procédé selon la revendication 1, **caractérisé en ce que** les produits d'addition de Michaël contenus dans le liquide F sont formés lors de la préparation d'un ester d'acide acrylique et d'un alcool en C₁-C₁₀ et le liquide F présente les teneurs suivantes :
| | |
|---|---|
| 1 à 30% en poids | d'esters de l'acide acrylique, |
| 40 à 80% en poids | de produits d'addition de Michaël ainsi que |
| au moins 15% en poids | de polymère radicalaire d'acide acrylique et/ou d'esters d'acide acrylique et |
| 0,1 à 2% en poids | d'inhibiteur de polymérisation. |

3. Procédé selon la revendication 1, **caractérisé en ce que** les produits d'addition de Michaël contenus dans le liquide F sont formés lors de la préparation d'acide acrylique et le liquide F présente les teneurs suivantes :
| | |
|---|---|
| 10 à 50% en poids | de produits d'addition de Michaël, |
| au moins 40% en poids | de polymère radicalaire d'acide acrylique, |
| jusqu'à 25% en poids | d'acide acrylique monomère |
| jusqu'à 2% en poids | d'inhibiteur de polymérisation, et |
| jusqu'à 15% en poids | d'autres composés. |

4. Procédé selon la revendication 1, **caractérisé en ce que** les produits d'addition de Michaël contenus dans le liquide F sont formés lors de la préparation d'acide acrylique et le liquide F présente les teneurs suivantes :
| | |
|---|---|
| 10 à 50% en poids | de produits d'addition de Michaël, |
| 40 à 80% en poids | de polymère radicalaire d'acide acrylique, |
| 5 à 20% en poids | d'acide acrylique monomère |
| 0,1 à 2% en poids | d'inhibiteur de polymérisation, et |
| 1 à 15% en poids | d'autre composé. |

5. Procédé selon la revendication 3 ou 4, **caractérisé en ce que** 40 à 60% en poids des produits d'addition de Michaël contenus dans le liquide F sont des dimères d'acide acrylique de Michaël et 15 à 30% en poids sont des trimères d'acide acrylique de Michaël.

6. Procédé selon l'une quelconque des revendications 1 à 5, **caractérisé en ce que** la colonne de séparation K contient des plateaux à double flux.

7. Procédé selon l'une quelconque des revendications 1 à 6, **caractérisé en ce que** l'échangeur thermique à circulation UW est exploité comme un échangeur thermique de détente à circulation forcée de manière telle qu'on trouve, entre le site d'alimentation II et la sortie du flux massique M dudit au moins un espace secondaire de l'échangeur thermique de détente à circulation forcée, un dispositif d'étranglement et la pression de travail, dans le sens d'écoulement, en amont du dispositif d'étranglement est supérieure à la pression gazeuse GD dans l'espace de fond.

8. Procédé selon l'une quelconque des revendications 1 à 7, **caractérisé en ce que**, pour soutenir le procédé de la dissociation en retour, un gaz de rectification est guidé dans la colonne de séparation K au-dessus du niveau S du liquide de fond et sous l'encastrement actif en séparation inférieur de la colonne de séparation K.

9. Procédé selon l'une quelconque des revendications 1 à 8, **caractérisé en ce que** la pression de travail en tête de la colonne de séparation K est de > 1 à 3 bars.

10. Procédé selon l'une quelconque des revendications 1 à 9, **caractérisé en ce que** l'échangeur thermique à circulation UW est un échangeur thermique à faisceau tubulaire.

11. Procédé selon la revendication 8, **caractérisé en ce que** ledit au moins un flux partiel II et le gaz de rectification sont guidés, au moyen d'un double tube coaxial constitué par un tube interne et un tube externe entourant celui-ci, dans l'espace de fond, le gaz de rectification étant guidé dans le tube interne et ledit au moins un flux partiel II étant guidé dans le tube externe et le tube interne est isolé thermiquement par rapport au tube externe.

12. Procédé selon la revendication 11, **caractérisé en ce que** tant le gaz de rectification que ledit au moins un flux partiel II s'écoulent du double tube coaxial sur un dispositif déflecteur qui dévie les deux flux vers le haut dans la colonne de séparation K.

13. Procédé selon l'une quelconque des revendications 1 à 12, **caractérisé en ce que** la viscosité dynamique du liquide de fond à la température T^{SU} est de 30 à 90.

14. Procédé selon l'une quelconque des revendications 1 à 13, **caractérisé en ce que** le taux d'efficacité Q du procédé de dissociation en retour est d'au moins 20%.
